# FASCICULE DE BREVET EUROPEEN

(11) **EP 1 885 721 B1**
(45) Date de publication et mention de la délivrance du brevet: **29.07.2009**
(21) Numéro de dépôt: 06725304.7
(22) Date de dépôt: 24.03.2006
(51) Int. Cl.: C07D 471/04, C07F 15/00, A61K 49/06, A61K 9/10

(54) **CHELATES MÉTALLIQUES DE DÉRIVÉS MACROCYCLIQUES POLYAMINOCARBOXYLIQUES ET LEUR UTILISATION EN IMAGERIE DIAGNOSTIQUE**
METALLCHELATE VON MAKROCYCLISCHEN POLYAMINOCARBONSÄUREDERIVATEN UND DEREN ANWENDUNG BEI DER DIAGNOSTISCHEN BILDERZEUGUNG
METAL CHELATES OF POLYAMINOCARBOXYLIC MACROCYCLIC DERIVATIVES AND USE THEREOF IN DIAGNOSTIC IMAGING

(30) Priorité: 24.03.2005 FR 0502921
(43) Date de publication de la demande: 13.02.2008
(73) Titulaire: GUERBET, 93420 Villepinte (FR)
(72) Inventeur: PORT, Marc, F-95170 Deuil La Barre (FR)
(74) Mandataire: Warcoin, Jacques
(86) Numéro de dépôt international: PCT/EP2006/061034
(87) Numéro de publication internationale: WO 2006/100305

(56) Documents cités:
- WO-A-00/75141
- WO-A-94/26315
- WO-A-03/074523
- WO-A-20/04112839
- AIME, SILVIO ET AL: "Designing novel contrast agents for magnetic resonance imaging. Synthesis and relaxometric characterization of three gadolinium(III) complexes based on functionalized pyridine-containing macrocyclic ligands" HELVETICA CHIMICA ACTA , 86(3), 615-632 CODEN: HCACAV; ISSN: 0018-019X, 2003, XP002355606
- DIOURY, FABIENNE ET AL: "Synthesis of a new tricyclic tetraazatriacetic acid as ligand for gadolinium(III)" TETRAHEDRON LETTERS , 46(4), 611-613 CODEN: TELEAY; ISSN: 0040-4039, 24 janvier 2005 (2005-01-24), XP004695928

## Description

L'invention concerne des composés chélates utilisables en IRM, les chélates étant destinés à être véhiculés par des transporteurs lipophiles tels que des nanoparticules lipidiques ou des liposomes. L'invention concerne aussi des composés comprenant en association ces chélates et ces transporteurs, reliés le cas échéant par des groupes chimiques de liaison, et leur utilisation en imagerie diagnostique, cette association pouvant en outre comprendre des marqueurs de ciblage biologique désignés biovecteurs.

On connaît déjà de nombreux chélates utilisés en IRM tels que des chélates d'ion de métal paramagnétique, notamment le DTPA, le DOTA, le DOTA bisamide, ainsi que des dérivés lipophiles de ces composés décrits dans les documents US 5 804 164, 5 460 799, WO91/14178. De tels dérivés sont décrits à la surface de liposomes ou sous forme de particules en émulsion par exemple dans les documents US 6 132 764, US 6 010 682, US 5 614 170, US 5 833 948. De telles compositions lipidiques et émulsions comprennent dans certains cas des molécules désignées biovecteurs destinés à la reconnaissance spécifique de zones d'intérêt diagnostique ou thérapeutique, notamment des zones pathologiques ayant une expression modifiée des molécules cibles de ces biovecteurs par rapport à des cellules normales. Des émulsions de nanoparticules de fluorocarbone comprenant des biovecteurs sont par exemple décrites dans WO 02/060524, WO 03/062198.

De nombreux chélates, rappelés par exemple dans le document US 20040248856, sont décrits ou seulement cités dans ces compositions de liposomes ou émulsions, notamment les chélates : DTPA, EDTA, DOTA, DTPA-BOA (bisamide), DOTA-PE, DTPA-PE (monoamide), ODDA, TTTA, DOTMA, DOTRP, DO3A, BOPTA.

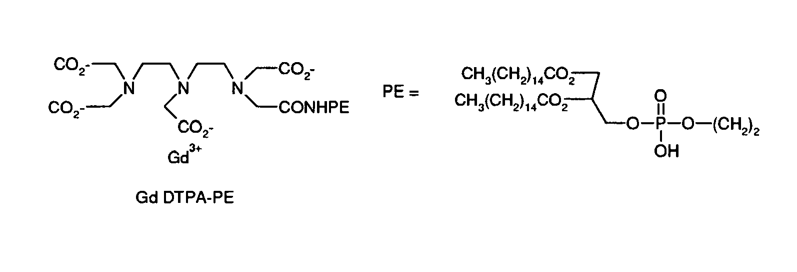

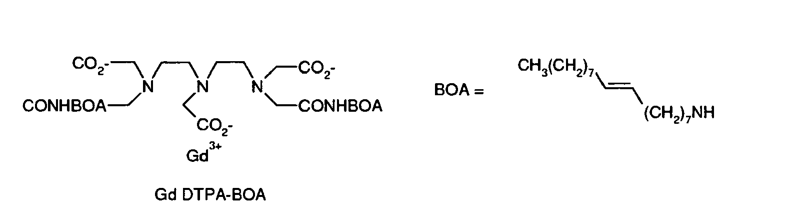

La technologie des micro/nano émulsions a permis de faire la preuve de concept de l'imagerie spécifique en T1 à des concentrations picomolaires (150pM) en nanoparticules (A. M. Morawski, P.M. Winter, S.D. Caruthers et Coll. Detection of angiogenic epitopes at picomolar concentrations with αvβ3-integrin targeted ultra paramagnetic nanoparticles in human cancer cells in vitro. Proc. Intl. Soc. Mag. Reson. Med. 2003,11, 831).
- en ciblage d'αvβα : angiogénèse tumorale à l'aide des anticorps DM 101 ou d'un antagoniste quinolone (WO 03 062198A) (modèles cornée de lapin, VX-2, mélanome C32, vasa vasorum de la plaque d'athérome (modèle New Zealand Rabbit à régime riche en cholestérol).
- en ciblage du facteur tissulaire (TF) à l'aide d'un anticorps polyclonal anti TF pour traiter la resténose (incorporation de doxorubicine dans la micro émulsion).
- en ciblage du thrombus à l'aide d'un anticorps monoclonal biotyné antifibrine (S. Flacke, S. Fischer, M.J. Scott et Coll. Novel MRI contrast agent for molecular imaging of fibrin : implications for detecting vulnerable plaques. Circulation 2001, 104, 1280-1285.).

Par ailleurs on connaît des dérivés de type PCTA, de formule 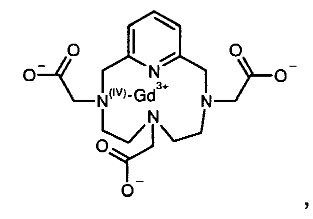 (WO 9426315 et US 6 440 956)
mais qui à la connaissance du demandeur présentent des chaînes courtes, ou des chaînes longues hydrophiles et non lipophiles. Les structures chimiques décrites ne permettent pas d'utiliser ces dérivés PCTA dans des compositions lipidiques en particulier de type émulsions, liposomes, micelles ou analogues.

Selon un premier aspect l'invention concerne de nouveaux composés de type chélates de type PCTA non décrits d'après le demandeur dans l'art antérieur pour une utilisation dans des compositions lipidiques.

L'invention concerne plus précisément des chélates lipophiles de formule (I) 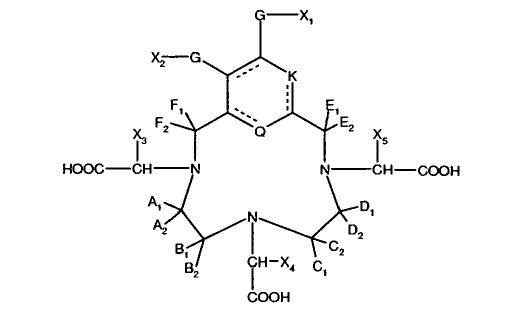 dans laquelle :
1) --- représente une simple ou double liaison ;
2) Q représente un atome d'azote ou un radical NH ;
3) X₁, X₂, X₃, X₄ et X₅, identiques ou différents, représentent indépendamment, à la condition que les X₁, X₂, X₃, X₄ et X₅ ne soient pas tous H et qu'au moins un des X₁, X₂, X₃, X₄, X₅ soit un groupe lipophile
   3.1) un atome d'hydrogène ;
   3.2) un groupe -(CH₂)ₐ-CONR₁R₂ , -(CH₂)ₐNR₁COR₂ ou 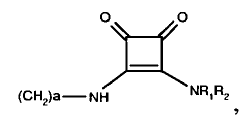 avec :
      a=1,2 ou 3;
      chaque R₁, R₂ représente indépendamment un atome H ; une chaîne alkyle ou alcényle en C₇-C₃₀, substituée ou non substituée, linéaire ou ramifiée ou cyclique, éventuellement interrompue par O, NH, NR₃ ou S, où R₃ est un alkyl en C₁-C₃ ; ou un groupe 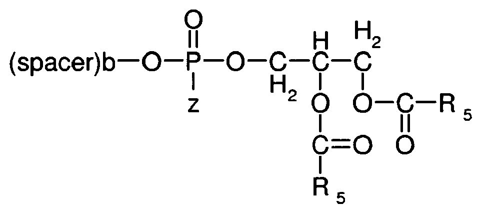 avec b = 0, 1 ou 2 , Z représente O⁻ ou OH, R₅ représente un groupe saturé ou insaturé d'au moins 6 atomes de carbone avantageusement en C₆ à C₃₀, éventuellement substitué, et spacer représente un groupe CH₂CH₂ ou polyalkylèneglycol ;
   3.3) un groupe 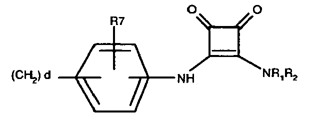 ou 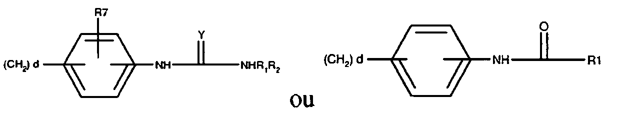 avec :
      d compris entre 0 et 3 ;
      Y choisi parmi O et S ;
      R₇ choisi parmi H, OH, CH₃, OCH_{3 ;}
      R₁ et R₂ sont tels que définis ci-dessus
4) A₁, A₂, B₁, B₂, C₁, C₂, D₁, D₂, E₁, E₂, F₁, F₂, représentent indépendamment les uns des autres un groupe H ou CH₃ ou cyclohexyle ;
5) K représente C ou N ou CH ou NH ou N⁺R₄ avec R₄ un groupe en C₁-C₆ choisi parmi alkyle, benzyle ou benzyle substitué ;
6) G représente rien ou O ou NHR₄, R₄ est tel que défini ci-dessus et ses sels pharmaceutiquement acceptables.

En particulier l'invention concerne un composé de formule (I) 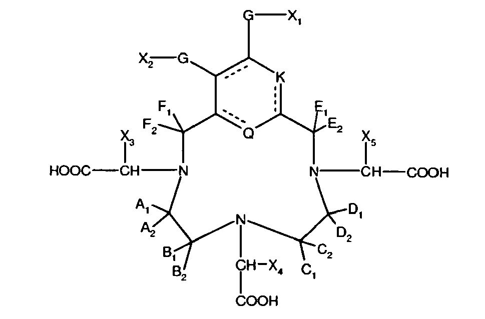 dans laquelle :
1) --- représente une simple ou double liaison
2) Q représente un atome d'azote ou un radical NH
3) X₁ ou X₂ identiques ou différents, représentent indépendamment un atome d'hydrogène, un groupe -(CH₂)ₐ-CONR₁R₂, -(CH₂)ₐ-NR₁-COR₂
   ou 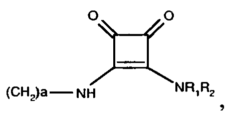 avec :
   a=1, 2 ou 3;
   chaque R₁, R₂ représente indépendamment un atome H, une chaîne alkyle ou alcényle en C₇-C₃₀, substituée ou non substituée, linéaire ou ramifiée ou cyclique, ou un groupe 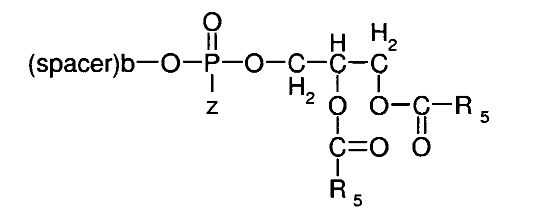 avec b = 0, 1 ou 2 , Z représente O⁻ ou OH, R₅ représente un groupe saturé ou insaturé d'au moins 6 atomes de carbone avantageusement en C₆ à C₃₀, avantageusement un groupe alkyle, éventuellement substitué, et spacer représente un groupe CH₂CH₂ ou polyalkylèneglycol ;
4) X₃, X₄ et X₅, identiques ou différents ont la même signification que X₁ ou X₂ à la condition que les X₁, X₂, X₃, X₄ et X₅ ne soient pas tous H et qu'au moins un des X₁, X₂, X₃, X₄, X₅ soit un groupe lipophile ou X₃, X₄ et X₅, identiques ou différents, représentent 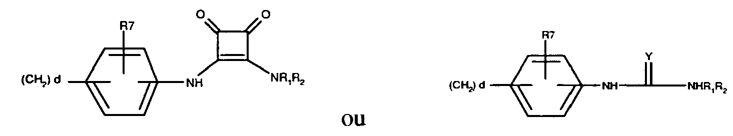 ou 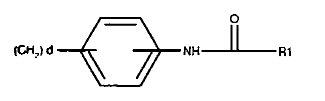 d compris entre 0 et 3 ;
   Y choisi parmi O et S ;
   R₇ choisi parmi H, OH, CH₃, OCH₃ ;
   R₁ et R₂ sont tels que définis ci-dessus ;
5) A₁, A₂, B₁, B₂, C₁, C₂, D₁, D₂, E₁, E₂, F₁, F₂, représentent indépendamment les uns des autres un groupe H ou CH₃ ou cyclohexyle ;
6) K représente C ou N ou CH ou NH ou N⁺R₄ avec R₄ un groupe en C₁-C₆ choisi parmi alkyle, benzyle ou benzyle substitué ;
7) G représente rien ou O ou NHR₄, R₄ est tel que défini ci-dessus et ses sels pharmaceutiquement acceptables.

Par l'expression « au moins un des groupes X₁, X₂, X₃, X₄, X₅ est un groupe lipophile », on entend que ces groupes X₁ à X₅ sont d'un caractère lipophile tel qu'ils permettent au composé (I) d'être lipophile.

Plus précisément, les groupes X₁ à X₅ sont tels que le composé (I) présente une lipophilie suffisante pour être couplé à un transporteur lipophile tel qu'un liposome, une micelle, une nanoémulsion. Il est précisé que le noyau PCTA du composé (I), en l'absence de chaîne X₁ à X₅, est hydrophile.

On aura par exemple, de manière non limitative, un choix des X₁ à X₅ tel que la valeur HLB (la balance hydrophile / lipophilie) du chélate (I) soit de l'ordre de 13 à 20 pour des chélates associés à des micelles directes ou à des nanoémulsions lipidiques, de l'ordre de 1 à 6 pour des chélates associés à des micelles inverses, de l'ordre de 6 à 8 pour des chélates associés à des liposomes. Ainsi, avantageusement, la valeur HLB du chélate (I) est comprise entre 1 et 20.

Avantageusement, seulement un ou deux des groupes X₁ à X₅ seront lipophiles, les autres groupes étant des chaînes courtes et non hydrophiles, tels que représentés dans les exemples 6 et 20 notamment.

Au sens de la présente invention, on entend par «groupe alcényle en C₇-C₃₀» tout radical hydrocarboné insaturé monovalent linéaire ou ramifié, contenant une double liaison et ayant de sept à trente atomes de carbone compris, à moins qu'il n'en soit autrement indiqué.

Selon une réalisation avantageuse, R₁ et/ou R₂ représentent une chaîne alkyle ou alcényle en C₇-C₂₄, de préférence en C₈-C₁₈, linéaire ou cyclique (par exemple comprenant un groupe phénylène, stéroide). De préférence R₁ et/ou R₂ représentent une chaîne alkyle ou alcényle linéaire en C₈-C₁₈. Par exemple, on peut citer comme exemples de chaînes X₁ à X₅ lipophiles des chaînes du type palmityle, oléyle, linoléyle, lauryle, stearyle, caproyle, capryle, caprylyle, arachidyle et analogues.

Dans le cas où les X₁ à X₅ représentent une chaîne alkyle ou alcényle en C₇-C₃₀ interrompus par O, NH, NR₃, les groupes X₁ à X₅ sont tels que le chélate de formule (I) soit lipophile. Avantageusement, dans ce cas, les chaînes alkyle ou alcényle en C₇-C₃₀ ne comprend pas plus de deux atomes ou groupes O, NH, NR₃.

Tout composé équivalent en terme d'activité biologique notamment aux composés lipophiles exemplifiés est toutefois dans le cadre de l'invention.

La présente invention concerne en outre un complexe d'un ion de métal paramagnétique et d'un chélate organique de formule (I) selon la présente invention.

Elle concerne également un complexe selon la présente invention dans lequel l'ion métallique est un lanthanide de nombre atomique 58-70 ou métal de transition de nombre atomique 21-29, 42 ou 44.

De plus, elle concerne un complexe selon la présente invention dans lequel l'ion métallique est choisi parmi Gd(III), Mn(II), fer, dysprosium.

Ces chélates et complexes métalliques lipophiles sont destinés à être associés à différents types de transporteurs lipophiles, le produit formé par l'association entre le transporteur et le chélate ou le complexe permettant de générer un signal en imagerie de diagnostic médical. Avantageusement, la relaxivité en IRM de ces chélates est meilleure que celle obtenue avec un noyau DOTA ou DTPA.

On peut aussi avoir K représenté par C-CH₂-O-R (avec R choisi parmi H, alkyle en C₁-C₅, benzyle, benzyle substitué) ou par C-COO-(alkyl en C₁-C₃), l'obtention de tels chélates étant décrite dans le document EP 579 802, à condition que ces groupes n'altèrent pas la lipophilie du chélate (I).

De manière plus large, on peut aussi avoir des groupes X₁ à X₅ s'écrivant (spacer)-Z, avec :
- spacer étant un groupe de liaison entre d'une part -CH- et un groupe lipophile autre que ceux exemplifiés dans la présente demande, le spacer comprenant un groupe capable de réagir avec une fonction du groupe lipophile (un très grand nombre de spacer peuvent être utilisés, à condition qu'ils ne modifient pas de manière gênante la lipophilie du chélate)
- Z étant tout groupe lipophile autre que ceux exemplifiés en détail dans la présente demande.

Selon un autre aspect l'invention concerne ainsi un composé comprenant un chélate de formule (I) ou un complexe (chélate couplé au métal) selon la présente invention couplé chimiquement avec un transporteur lipophile approprié, choisi de préférence parmi les liposomes, les nanoparticules de fluorocarbone, les émulsions d'huile, les micelles. L'invention concerne également les compositions de diagnostique, en particulier les agents de contraste comprenant ces composés ou complexe ou composition.

Ces chélates sont capables de chélater typiquement des ions de métaux paramagnétiques pour l'IRM, des radionucléides pour la scintigraphie et la TEP (tomographie par émission de positons). Le signal permet la mesure d'un paramètre quantifié notamment en IRM (relaxivité) ou en scintigraphie ou en TEP.

Par l'expression composé comprenant un chélate de formule (I), on inclut le cas de l'utilisation de différents chélates de formule (I) dans une même composition lipidique administrée au patient.

La présente invention concerne en outre une composition lipidique physiologiquement acceptable destinée à l'imagerie IRM comprenant au moins un complexe selon la présente invention.

Elle concerne également une composition selon la présente invention sous forme d'émulsion, de liposomes, de micelles.

De plus, elle concerne une composition selon la présente invention comprenant de l'eau, une phase lipidique dispersée, éventuellement un fluorocarbone, et au moins un complexe selon la présente invention.

Selon une réalisation ces transporteurs sont associés d'une part à au moins un chélate de formule (I) et d'autre part à au moins un biovecteur de ciblage spécifique du diagnostique d'une pathologie, notamment dans les domaines des maladies cancéreuses, des maladies inflammatoires et neurodégénératives, des maladies cardiovasculaires.

On pourra notamment utiliser les composés suivants :

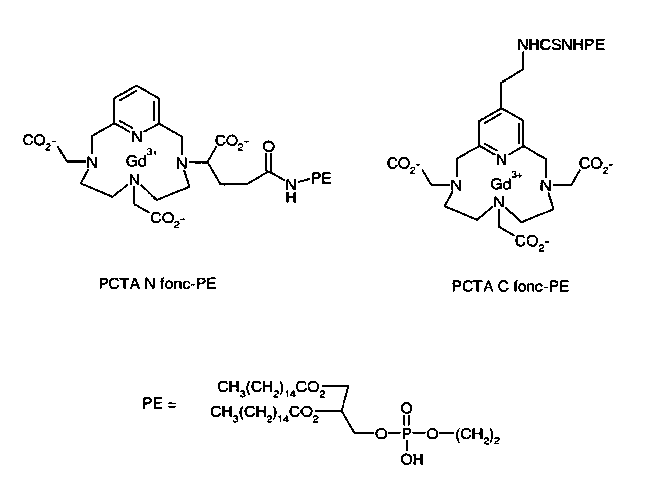

Par chélate lipophile on entend que le chélate a été modifié chimiquement de manière à présenter une lipophilie (une lipophilie suffisamment élevée, ou à l'inverse une hydrophilie suffisamment faible), telle qu'il peut être associé avec le transporteur lipophile de manière à former une composition lipidique suffisamment stable pour une utilisation diagnostique satisfaisante.

Les métaux paramagnétiques incluent les lanthanides de nombre atomique 58-70 et les métaux de transition de nombre atomique 21-29, 42 or 44, par exemple scandium, titanium, vanadium, chromium, manganèse, fer, cobalt, nickel, cuivre, molybdenum, ruthenium, cerium, praseodymium, neodymium, promethium, samarium, europium, gadolinium, terbium, dysprosium, holmium, erbium, thulium, et ytterbium. On préfère particulièrement les éléments Gd(III), Mn(II), europium, dysprosium. Les radionucléides incluent les formes radioactives des éléments Sm, Ho, Y, Pm, Gd, La, Lu, Yb, Sc, Pr, Tc, Re, Ru, Rh, Pd, Pt, Cu, Au, Ga, In, Sn, Cr, Pb.

Selon une réalisation, comme dans le document US 20040248856, on utilise comme groupe lipophile du chélate de formule (I) des phosphoglycerides issue typiquement de lécithines dans lesquelles les groupes R₅COO sont des acides gras tels que les acides oléique, palmitique, stéarique. On peut aussi utiliser des phosphoglycérides où R₅ est un groupe optionellement substitué hydrocarbyle saturaté ou insaturaté. Le groupe hydrocarbyle contient typiquement au moins 6, de préférence au moins 10 atomes de carbone pour une lipophilie suffisante. Le groupe R₅ peut inclure un ou plusieurs groupes cycliques.

On utilise ou non un groupe spacer, le spacer pouvant inclure une partie issue du phosphoglycéride, par exemple un groupe CH₂CH₂ issu d'un phosphodiglycéride de type phosphatidyl éthanolamine.

Selon des réalisations le spacer inclut des parties dérivées de peptides, pseudopeptides, enchaînements d'acides aminés, polyalkylene glycols, notamment de polyethylene glycol PEG et analogues.

Les composés de formule (I) sous forme de complexe selon la présente invention sont inclus dans des compositions contenant des transporteurs lipophiles, ces compositions se présentant sous la forme de particules lipophiles au moins en surface, mises en suspension dans un milieu aqueux ou hydrophile. Ces particules sont des nanoparticules, de diamètre de l'ordre de 10 nm à 500 nm, de préférence 10 à 100, voire 10 à 50 nm.

On citera comme particules possibles des liposomes, unilamellaires ou multilamellaires, des micelles, des gouttelettes d'huiles, des lipoprotéines, tels que HDL, LDL, IDL, VLDL, chylomicrons, des nanoparticules de fluorocarbone, des nanobulles, ou analogues dont la surface est lipophile.

Par particule, le nombre de chélate est de l'ordre de 20 000 à 100 000 pour les nanoémulsions (diamètre hydrodynamique > 80 nm), de l'ordre de 200 à 15 000 pour les liposomes, de l'ordre de 10 à 500 pour les micelles.

Dans le cas de transporteurs lipidiques de type liposomes, on les préparera par exemple à l'aide de phospholipides issus de la choline (phosphatidylcholines), la serine (phosphatidylserines), du glycerol (phosphatidylglycerols), l'ethanolamine (phosphatidylethanolamines), de l'inositol (phosphatidylinositol). Les acides gras utilisés seront par exemple des chaînes aliphatiques comprenant 10 à 24 atomes de carbone. On utilisera notamment des acides gras suivants : laurique, myristique, palmitique, stéarique, oléique, linoleéique, notamment les acides gras saturés. Parmi les dérivés phospholipides utilisables connus on citera les suivants : (DLPC), (DMPC), (DPPC), (DAPC), (DSPC), (DOPC), (DPDPC), (PMPC), (DLPG), (DPPG). On pourra aussi utiliser des phosphatidyléthanolamines et leurs dérivés avec des PEG de poids moléculaires variés (300 à 5000 daltons), tels que le DPPE-PEG (DSPE-PEG), par exemple le DPPE-PEG2000. De nombreux procédés de préparation de liposomes intégrant des chélates sont connus, notament dans WO92/21017. Par exemple, dans le cas de phospholipides on réalise la dissolution des phospholipides dans un solvant organique, l'évaporation du solvant sous vide pour obtenir un film des composés formant le liposome, l'hydratation du film. La taille du liposome est obtenue par différentes techniques possibles telles que la sonication, l'extrusion ou la microfluidisation de la suspension initiale de liposome. Les composés (I) seront typiquement intégrés dans la bicouche lipidique des liposomes.

Pour les micelles on pourra utiliser en particulier ceux décrits dans "Surfactants and Polymers in Drug Delivery", by M. Malmsten, Ch. 2, pp. 19-50, Marcel Dekker Inc. Ed.2002 ; et notamment des micelles à base de phospholipides et de dérivés PEG-phospholipides. On pourra aussi utiliser des associations de micelles et de liposomes en fonction de leurs charges, une micelle pouvant comprendre plusieurs centaines de molécules chélates.

Selon des réalisations ces transporteurs comprennent des constituants de type surfactants qui sont utiles notamment pour le couplage avec des biovecteurs grâce à des groupements fonctionnels appropriés des constituants lipides/surfactants (phosphatidyléthanolamine par exemple). De multiples possibilités de couplage sont rappelées dans US 20040248856.

Pour des compositions destinées au ciblage spécifique, on peut utiliser les biovecteurs notamment rappelés dans le document WO 2004/112839 (en particulier pages 60 à 82). On citera notamment des peptides, des ligands de reconnaissance spécifique de récepteurs, des dérivés de vitamines, notamment pour le ciblage d'intégrines, de fibrine, de récepteurs EGF/VEGF. On pourra utiliser des ligands amphiphiles capables de se mélanger avec des composants des micelles, ou des ligands amphiphiles aptes à se lier à des composants amphiphiles des liposomes ou des micelles. Par exemple le ligand peut se lier à des composants amphiphiles des liposomes ou des micelles par l'intermédiaire de fonctions acide, isothiocyanate. Le ligand peut être incorporé lors de la préparation des micelles, ou fixé aux micelles déjà préparées et incorporant des groupes amphiphiles destinées à être couplées au ligand.

Selon des réalisations avantageuses pour la préparation de nanoémulsions lipidiques, on utilise des liquides de type perfluorocarbone tels que décrits dans US 5,958,371, l'émulsion liquide contenant des nanoparticules comportant un perfluorocarbone à point d'ébullition assez élevé (par exemple entre 50 et 90 °C) entouré d'un revêtement composé d'un lipide et/ou d'un surfactant. Le surfactant est capable de se coupler directement à un biovecteur de ciblage ou d'inclure un composé intermédiaire lié de manière covalente à au biovecteur, le cas échéant à l'aide d'un agent de liaison chimique. On peut aussi utiliser un revêtement cationique de manière à adsorber des biovecteurs chargés négativement. On citera comme exemple de lipides/surfactants des phospholipides, des acides gras, des cholestérol, des lysolipides, des sphingomyélines, des lipides conjugués à des PEG.

On utilisera alors par exemple comme phospholipides les composés suivants : phosphatidylcholine dioléoylphosphatidylcholine, dimyristoylphosphatidylcholine, dipalmitoylphosphatidylcholine, distéaroylphosphatidylcholine, phosphatidyléthanolamine.

On utilisera alors par exemple des perfluorocarbones choisis parmi : perfluorodécalin, perfluorooctane, perfluorodichlorooctane, perfluoro-n-octyl bromide, perfluoroheptane et analogues.

Selon des réalisations avantageuses, on utilisera pour préparer des agents de contraste selon l'invention des méthodes et des compositions lipidiques appropriées notamment des liposomes, des micelles, des émulsions.

On rappelle que les nanoémulsions sont des mélanges lipidiques hétérogènes de lipides obtenues de manière appropriée par agitation mécanique et/ou addition d'agents émulsifiants. Par exemple on mélange mécaniquement les chélates rendus lipophiles à des solvants organiques tels que le chloroforme. Après évaporation du solvant, les lipides sont remis en suspension en milieu aqueux tel que PBS pour obtenir une émulsion qui subit ensuite typiquement une sonication et une microfluidisation. Les émulsions obtenues peuvent être lyophilisées avec le cas échéant utilisation d'agents anti agglutination.

La présente invention concerne donc un procédé de préparation d'une composition selon la présente invention comprenant :
- la préparation d'un mélange comprenant un complexe selon la présente invention et une phase aqueuse ;
- l'agitation du mélange de manière à obtenir une dispersion homogène des constituants.

Selon une autre réalisation, on prépare les émulsions selon le procédé suivant :
mélange :
- phase huileuse de perfluorocarbone (40% environ)
- eau + huile de carthame et/ou glycérol
- solution de tensioactif (1 à 10%, de préférence 2 à 5%, cette solution étant un mélange comprenant par exemple le chélate lipophile, du cholestérol, un phospholipide tel que la lécithine, et éventuellement un biovecteur). La solution de tensioactif contient typiquement 10 à 50 % de chélate ou complexe selon l'invention.
-le cas échéant tensioactif fluoré, notamment à fort HLB tel que Pluronic F 68, le Docécyl Sulfate de Sodium, le Triton, le Tween.

De manière très avantageuse on utilisera des micelles (monocouches lipidiques, les liposomes étant des bicouches lipidiques) :
- dont la taille est contrôlable de manière plus facile que celle de liposomes, la taille préférée des micelles étant de l'ordre de 30 à 300 nm, de préférence 50 à 100 nm, ce qui leur donne un accès plus facile aux tissus que des particules plus grosses
- qui sont utilisables avec ou sans incorporation de gaz, les gaz étant utiles notamment en imagerie par ultrasons mais pas nécessairement en IRM
- qui peuvent contenir une phase huileuse qui ne pose pas de problème de stabilité, de manière à avoir une stabilité de plusieurs mois.

De plus dans le cas des micelles les chélates s'agencent plus aisément à la périphérie externe de la monocouche, ce qui est avantageux si l'on souhaite éviter l'incorporation de chélates à l'intérieur de la sphère formée par le micelle, et optimise le signal pour une quantité de chélates donnée de la composition.

Pour formuler l'émulsion d'agent de contraste paramagnétique souhaité, on utilise typiquement 1 à 75% en poids de composé chélate lipophile ou de complexe selon l'invention par rapport aux ingrédients totaux de l'émulsion. La composition formant l'agent de contraste est administrée de préférence en intravasculaire, selon le patient examiné, par exemple à raison de 0,1 mg à 1 g de composé chélate lipophile et de 1 à 50 micromoles d'ion de métal paramagnétique par kg de patient.

Les compositions lipidiques obtenues sont le cas échéant formulées à l'aide d'additifs notamment pour une administration par injection intraveineuse. On citera notamment le dextrose, le chlorure de sodium, des antimicrobiens.

Avantageusement grâce aux compositions selon l'invention on peut obtenir une augmentation de la relaxivité par ion. On obtient typiquement des relaxivités r1 de l'ordre de 10 à 30 s⁻¹Gd⁻¹ voire davantage aux champs de 0,5 à 1,5 T environ. Le couplage approprié de chélates de formule (I) ou de complexe selon l'invention avec les particules est destiné à obtenir une association d'un nombre très élevé de chélates, de l'ordre de 2000 à 100 000 chélates par particule, typiquement de 10 000 à 50 000 chélates. On peut ainsi obtenir des particules dont les caractéristiques principales sont par exemple les suivantes, ces caractéristiques pouvant varier selon les compositions précises des émulsions, leur procédé de préparation, la nature du biovecteur :
- index de polydispersité : 0,2 à 0,3 ;
- [Gd³⁺] = 2 à 10 mM de préférence 3 à 7 mM ;
- concentration en particules : 50 à 100 nM ;
- r1 (mM⁻¹s⁻¹Gd⁻¹) : 5 à 40, de préférence 10 à 40 ;
- r2 (mM⁻¹s⁻¹Gd⁻¹) : 20 à 40 ;
- r1 (mM⁻¹s⁻¹particule⁻¹) : 10⁶ à 4x10⁶ ;
- nombre de biovecteurs : 50 à 1000, notamment 100 à 300 ;

En plus des dérivés PCTA, le demandeur a également étudié :
- des chélates lipophiles de type TRITA PE et GdP8A-PE 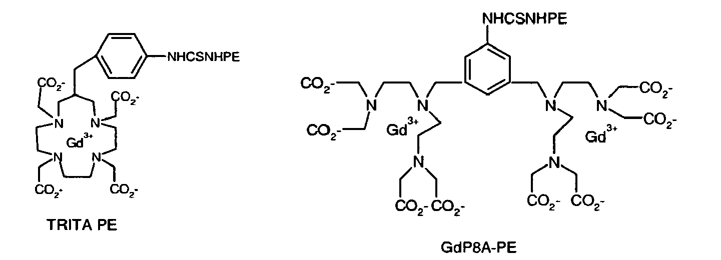 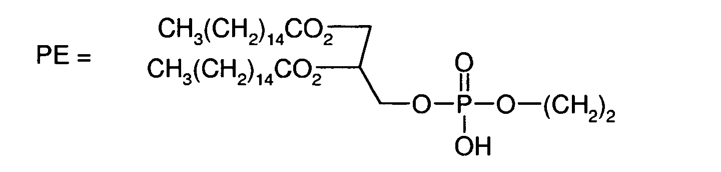
- des chélates lipophiles comprenant au moins une partie lipophile couplée à d'autres chélates par exemple choisis parmi les suivants :
   - chélates dérivés du D03A dont HDD-D03A, tels que décrits dans les documents: Magnetic Resonance Materials in Physics, Biology and Medicine, 12, 2001, 114-120 ; J.Chem.Soc, Perkin Trans, 2, 2001, 929-933 ; Academic Radiology, vol 9, suppl 1, 2002 ; Chem.EurJ, 1995, 10, 2977-2983
   - chélates décrits dans les documents W02004/087656, WO 03/008390, US 2003/171561 (Tweedle), US 6 719 958, US 2002/090342, US 6 517 814.

L'invention concerne aussi les compositions lipidiques comprenant en association au moins un chélate de formule (I) et au moins un chélate lipophile de l'art antérieur cité dans la demande. De plus on pourra utiliser plusieurs biovecteurs différents dans une même composition diagnostique, par exemple lorsque plusieurs biovecteurs différents sont capables de cibler une même pathologie.

L'invention concerne aussi les composés intermédiaires de formule (II) pour la préparation d'un composé de formule (I) 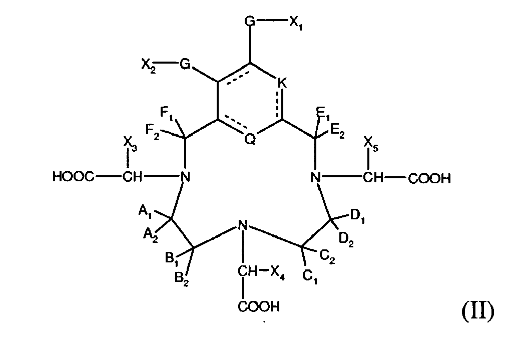 dans laquelle :
1) --- représente une simple ou double liaison ;
2) A₁, A₂, B₁, B₂, C₁, C₂, D₁, D₂, E₁, E₂, F₁, F₂, Q, K, R₇ ont la même signification que dans les revendications précédentes, G représente rien ; et X₁, X₂, X₃, X₄ ou X₅ identiques ou différents, représentent indépendamment (CH₂)a-NH₂ ou (CH₂)a-NO₂ ou 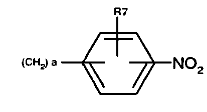 avec a=1, 2 ou 3
   ou A₁, A₂, B₁, B₂, C₁, C₂, D₁, D₂, E₁, E₂, F₁, F₂, Q, K, R₇ ont la même signification que dans les revendications précédentes, G représente O ou NHR₄ dans lequel R₄ a la même signification que dans les revendications précédentes et X₁, X₂, X₃, X₄ ou X₅ identiques ou différents représentent indépendamment (CH₂)a-CO₂H, ou 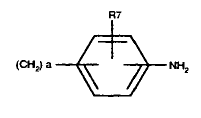
ou (CH₂)a-NH₂ ou (CH₂)a-NO₂ ou 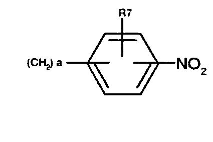 avec a=1, 2 ou 3.

Avantageusement X₁ ou X₂ identiques ou différents, représentent indépendamment (CH₂)a-CO₂H ou (CH₂)a-NH₂ ou (CH₂)a-NO₂.

D'autres aspects de l'invention sont illustrés dans les exemples qui suivent, sachant que les composés des exemples 5, 6, 7, 8, 10, 17, 18, 19, 20, 21, 22 sont des chélates lipophiles porteurs de chaîne lipophile selon l'invention.

### EXEMPLE 1

### a) N,N"-bis(o-nitrophénylsulfonyl)diéthylènetriamine

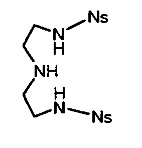

On dissout 8,4 g de NaOH dans 100 ml d'H₂O à 0° C et on ajoute 9,2 g de diéthylènetriamine, puis goutte à goutte à 0° C une solution de 41,5 g du chlorure de l'acide 2-nitrophénylsulfonique dissous dans 100 ml de tétrahydrofuranne.

Le milieu réactionnel est repris dans CH₂Cl₂ et la phase organique est lavée par H₂O puis séchée sur sulfate de magnésium. Après évaporation du solvant, on obtient 37,5 g de cristaux.
m/z : ES+ 474,7

### b) N,N"-bis(2-nitrophénylsulfonyl)N'-t-butoxycarbonyl diéthylène triamine

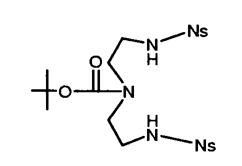

18 g de carbonate de di-tert-butyle sont ajoutés par fraction à une solution contenant 32,4 g de composé obtenu à l'étape a) dans un mélange de 97 ml de solution aqueuse de NaOH 2N et 225 ml de CH₃CN.

Après 3 h d'agitation à 25° C, le milieu réactionnel est évaporé à sec et le résidu est repris par 400 ml de CH₂Cl₂. La phase organique est lavée 2 fois avec 100 ml d'H₂O.

Après séchage sur sulfate de magnésium puis concentration de la phase organique, le résidu obtenu est purifié par chromatographie sur colonne (d = 15 cm) contenant 1 kg de silice (Merck^{®} 40-63 µm) en éluant avec un mélange CH₂Cl₂/CH₃COCH₃ gradient de 99/1 à 90/10 (v/v).

Après évaporation du solvant, on obtient 28,5 g de produit.
m/z : ES- 572,5

### c) Ester tert-butylique de l' acide 3,9-Bis-(2-nitro-benzènesulfonyl)-3,6,9,15-tétraaza-bicyclo[9.3.1]pentadéca-1(14),11(15),12-triène-6-carboxylique

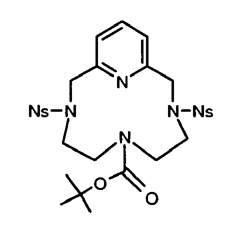

Une solution contenant 28 g du composé obtenu à l'étape b) dans 210 ml de CH₃CN en présence de 41,4 g de K₂CO₃ calciné est portée à reflux pendant 1 h 30.

Après addition de 11 g de 2,6-bis-(chlorométhyl)-pyridine, le mélange est chauffé à reflux pendant une nuit.

Le précipité formé est filtré, lavé avec 11 d'eau, puis séché sous vide ; m = 26,8 g.
m/z : ES+ 677,8

### d) Ester tert-butylique de l'acide 3,6,9,15-Tétraaza-bicyclo[9.3.1]pentadéca-1(14),11(15),12-triène-6-carboxylique

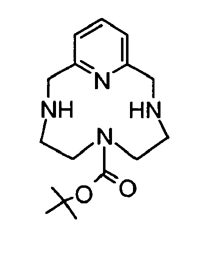

A 20 g du composé obtenu à l'étape c) en suspension dans 310 ml de diméthylformamide, on ajoute 16 g de LiOH puis goutte à goutte, en ½ h, 18 g d'acide thioglycolique.

Le milieu réactionnel coloré en rouge est agité pendant 6 h à 25° C puis 400 ml d'H₂O et 400 ml de CH₂Cl₂ y sont ajoutés. Après agitation et décantation, la phase aqueuse est séparée et extraite par 400 ml de CH₂Cl₂. Cette phase organique, après lavage deux fois à l'eau, est réunie à la phase précédente et l'ensemble est concentré. Le résidu huileux est purifié par chromatographie flash sur silice (Merck^{®}, 40-63 µm) en éluant avec un mélange CH₃OH/NH₄OH (50/1) après élimination des impuretés par élution avec CH₃OH.

Après évaporation des fractions conformes, on obtient 5,5 g du produit.
m/z : ES+ 307,6

### e) Ester tert-butylique de l'acide 3,9-Bis-éthoxycarbonylméthyl-3,6,9, 15-tétraaza-bicyclo[9.3.1]pentadéca-1(14),11(15),12-triène-6-carboxylique

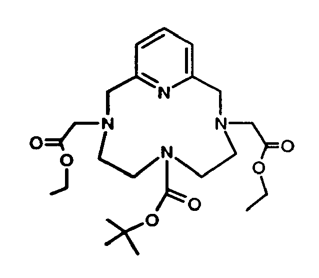

A une solution de 8 g de composé obtenu à l'étape d) dans 60 ml de CH₃CN et 26 ml d'éther diisopropylique, on ajoute 9,6 g de bromoacétate d'éthyle et 11 g de K₂CO₃ calciné puis le mélange est porté à sa température de reflux pendant 24 h.

Après filtration puis évaporation sous vide, l'huile obtenue est purifiée par flash-chromatographie sur silice (Merck^{®} , 40-63 µm) en éluant avec un mélange heptane/acétate d'éthyle (60/40 v/v).

Après évaporation, on obtient 6g d'huile.
m/z : ES+ 479.4

### f) Ester éthylique de l'acide(9-Ethoxycarbonylméthyl-3,6,9,15-tétraaza-bicyclo[9.3.1]pentadéca-1(15),11,13-trièn-3-yl)-acétique

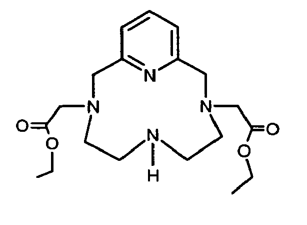

5 g du composé obtenu à l'étape e) sont dissous dans 30 ml d'acide trifluoroacétique et le mélange est agité durant 1 h 30 à 25° C.

Après concentration sous vide du milieu réactionnel, l'huile obtenue est purifiée par flash-chromatographie sur silice (Merck^{®} , 40-60 µm) en éluant avec un mélange CH₂Cl₂/CH₃OH (97/3, v/v).

Après élimination du solvant, on obtient 3 g du produit solide.
m/z : ES+ 379.5

### g) Ester méthylique de l'acide 2-(3,9-Bis-éthoxycarbonylméthyl-3,6,9,15-tétraaza-bicyclo[9.3.1]pentadéca-1(14),11(15),12-trièn-6-yl)-4-(4-nitro-phényl)-butyrique

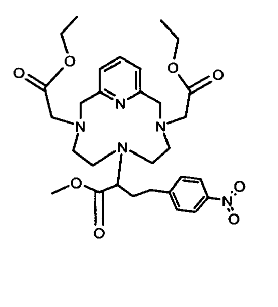

A une suspension de 3 g du composé obtenu à l'étape f) dans 30 ml d'éther diisopropylique et 65 ml de CH₃CN en présence de 2,5 g de K₂CO₃ calciné, on ajoute 3,6 g de l'ester méthylique de l'acide 2-Bromo-4-(4-nitro-phenyl)-butyrique. Après agitation 48 h à 85° C, le milieu réactionnel est filtré et concentré sous vide, l'huile résiduelle est purifiée par chromatographie sur colonne silice (Merck^{®} 40-60 µm) en éluant avec un mélange CH₂Cl₂/acétone 70/30 v/v.

Après concentration à sec, on obtient 2g de produit.
m/z : ES+ 600,5

### h) Acide 2-(3,9-Bis-carboxyméthyl-3,6,9,15-tétraaza-bicyclo[9.3.1]pentadéca-1(14),11(15),12-trièn-6-yl)-4-(4-nitro-phényl)-butyrique

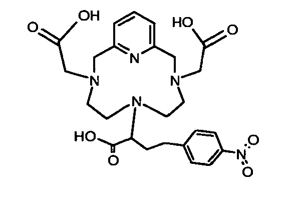

2 g du composé obtenu à l'étape g) sont ajoutés à une solution de 10 ml d'HCl 12N, puis le mélange est agité 48 h à sa température de reflux.

Après filtration, concentration, le résidu est purifié par chromatographie sur gel de silice silanisée (Merck^{®} 0,063-0,20 µm) en éluant avec un mélange H₂O/CH₃OH pour donner 1,5 g de produit.
m/z : ES- 528,4

### i) Complexe de gadolinium de l'acide 2-(3,9-Bis-carboxyméthyl-3,6,9,15-tétraaza-bicyclo[9.3.1]pentadéca-1(14),11(15),12-trièn-6-yl)-4-(4-nitro-phényl)-butyrique

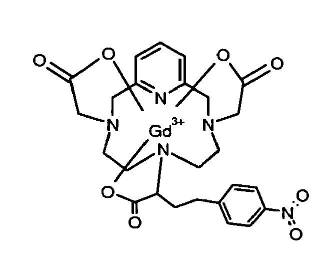

On introduit dans 40 ml d'H₂O, 1g du composé obtenu à l'étape h).

Le pH de la suspension est amené à 5 par ajout d'une solution de NaOH aqueuse 2N puis le milieu est chauffé à 50° C jusqu'à solubilisation complète.

Après addition de 350mg de Gd₂O₃, la solution toujours maintenue à pH 5 par ajout d'une solution aqueuse de NaOH 2N, est chauffée à 80° C pendant 6 h.

Après filtration des sels par évaporation, le résidu est cristallisé dans l'éthanol; le précipité est dissous dans l'eau et traité par une résine Chelex^{®} 100 (Bio-Rad). Après précipitation dans l'éthanol le précipité est filtré et séché. m = 1,2 g.
m/z : ES- 682,9

### j) Complexe de gadolinium de l'acide 4-(4-Amino-phényl)-2-(3,9-bis-carboxyméthyl-3,6,9,15-tétraaza-bicyclo[9.3.1]pentadéca-1(14),11(15),12-trien-6-yl)-butyrique

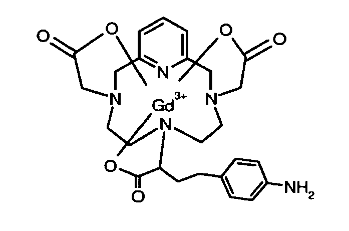

A 1 g du composé obtenu à l'étape i) dissous dans 50 ml d'H₂O, on ajoute 0,4 g de catalyseur charbon palladié à 10%, puis le milieu réactionnel est agité à 25° C sous une pression d'hydrogène de 3.10⁵ Pa pendant 6 h. Après élimination du catalyseur par filtration sur filtre Millipore^{®} (0,45 µm et 0,22 µm), la solution est évaporée pour donner 0,8 g de produit.
m/z : ES+ 654,9

### EXEMPLE2

### a) Ester méthylique de l'acide 4-(4-Nitro-phényl)-2-(3,6,9,15-tétraaza-bicyclo[9.3.1]pentadéca-1(14),11(15),12-trien-3-yl)-butyrique

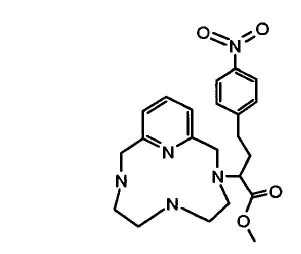

A une suspension de 70 g de 3,6,9-15-tétraazabicyclo[9.3.1.]pentadéca-1(15),11,13-triène dans 800 ml de CH₃CN en présence de 910 ml de résine échangeuse d'anions sous forme de base forte (Amberlite^{®} IRA458), on ajoute une solution de 102 g de l'ester 2-bromo-4-nitrophényl butyrate de méthyle dans 100 ml de CH₃CN.

Après agitation 3 jours à 25° C, filtration de la résine et évaporation, l'huile obtenue est purifiée par chromatographie sur colonne de 5 kg de silice (Merck^{®}, 40-60 µm) en éluant avec un mélange CH₂Cl₂/CH₃OH (70/30 v/v). On obtient 38 g de produit.
m/z : ES+ 428,6

### b) Ester méthylique de l'acide 2-(6,9-Bis-éthoxycarbonylméthyl-3,6,9,15-tétraaza-bicyclo[9.3.1]pentadéca-1(14),11(15),12-trièn-3-yl)-4-(4-nitro-phényl)-butyrique

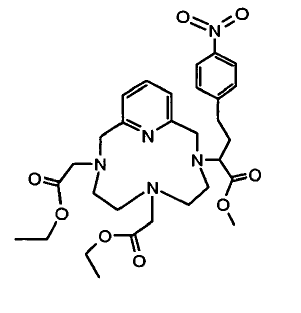

A une solution de 5 g du composé obtenu à l'étape a) dans 70 ml de CH₃CN et 35 ml d'éther diisopropylique, on ajoute 5 g de K₂CO₃ et 4,3 g de bromoacétate d'éthyle puis on laisse sous agitation 24 h à reflux.

Après élimination des sels par filtration, concentration de la solution, l'huile obtenue est purifiée par chromatographie sur silice (Merck^{®} 40-63 µm) en éluant avec un mélange CH₂Cl₂/acétone (70/30 v/v).

On obtient 4,9 g de produit solide.
m/z : ES+ 600,6

### c) Acide 2-(6,9-Bis-carboxyméthyl-3,6,9,15-tétraaza-bicyclo[9.3.1]pentadéca-1(14),11(15),12-trièn-3-yl)-4-(4-nitro-phényl)-butyrique

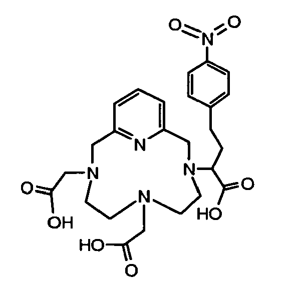

En appliquant le même mode opératoire que pour l'étape h) de l'exemple 1, on obtient 2,8 g de produit à partir de 4 g du composé obtenu à l'étape b).
m/z : ES- 528,4

### d) Complexe de gadolinium de l'acide 2-(6,9-Bis-carboxyméthyl-3,6,9,15-tétraaza-bicyclo[9.3.1]pentadéca-1(14),11(15),12-trien-3-yl)-4-(4-nitro-phényl)-butyrique

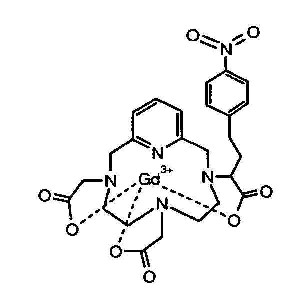

Dans 30 ml d'une solution à pH 5 de 2 g du composé obtenu selon l'étape c), on introduit 1,4 g de GdCl₃, 6H₂O et on maintient le mélange à 50° C pendant 5 h au cours desquelles on ajuste le pH en ajoutant une solution de NaOH aqueux (2N).

Le milieu est ensuite filtré puis évaporé ; 4 g de résine échangeuse de cations faiblement acide Chelex^{®} 100 (Bio-Rad) sont ajoutés à l'huile obtenue dissoute dans 40 ml d'eau.

Après agitation 2 h à 25° C, la résine est éliminée par filtration, la solution est évaporée pour donner 2,3 g de produit.
m/z : ES- 682,9

### e) Complexe de gadolinium de l'acide 4-(4-Amino-phényl)-2-(6,9-bis-carboxyméthyl-3,6,9,15-tétraaza-bicyclo[9.3.1]pentadéca-1(14),11(15),12-trièn-3-yl)-butyrique

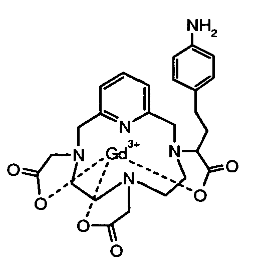

En appliquant le même mode opératoire que pour l'étape j) de l'exemple 1, on obtient 1,8 g de produit à partir de 2 g du composé obtenu à l'étape d).
m/z : ES+ 654,7

### EXEMPLE 3

### a) Ester diéthylique de l'acide 2-(3,9-Bis-éthoxycarbonylméthyl-3,6,9,15-tétraaza-bicyclo[9.3.1]pentadéca-1(14),11(15),12-trien-6-yl)-pentanedioique

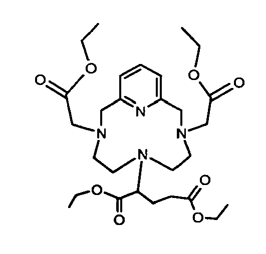

En appliquant le même mode opératoire que pour l'étape g) de l'exemple 1 on obtient 1.2g de produit au départ de 2 g de composé obtenu à l'étape f) de l'exemple 1) et 3 g de bromoglutarate de diéthyle après chromatographie sur colonne silice (Merck^{®} 40-60 µm) en éluant avec un mélange CH₂Cl₂/acétone 70/30 v/v.
m/z: ES+ 565,5

### b) Acide 2-(3,9-Bis-carboxyméthyl-3,6,9,15-tétraaza-bicyclo[9.3.1]pentadéca-1(14),11(15),12-trien-6-yl)-pentanedioique

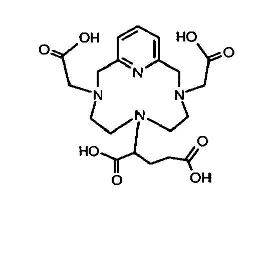

En appliquant le même mode opératoire que pour l'étape h) de l'exemple 1, on obtient 1,7g de produit à partir de 3 g du composé obtenu à l'étape a) après chromatographie sur silice silanisée.
m/z : ES- 451.6

### c) Complexe de gadolinium de l'acide 2-(3,9-Bis-carboxyméthyl-3,6,9,15-tétraaza-bicyclo[9.3.1]pentadéca-1(14),11(15),12-trièn-6-yl)-pentanedioique

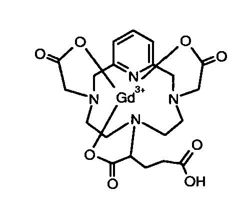

En appliquant le même mode opératoire que pour l'étape i) de l'exemple 1, on obtient 1,2 g de produit à partir de 1 g du composé obtenu à l'étape b).
m/z : ES- 605,5

### EXEMPLE 4

### a) 3-hydroxy-2,4-bis(hydroxyméthyl)pyridine :

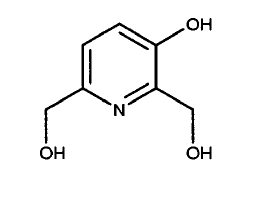

71,1g (0.75mol) de 3-hydroxypyridine sont dissous dans 300mL d'une solution aqueuse de NaOH à 10%. L'ensemble est porté à 90°C puis on introduit par portions 7x60mL d'une solution de formaldéhyde à 30% (5,mol). Laisser la nuit à température ambiante puis neutraliser par CH₃COOH. Concentrer et reprendre dans 600mL de DMF. Séparer le léger insoluble. Acidifier les jus par 75mL d'HCl 10N.

Concentrer. Purification de l'huile résiduelle par chromatographie sur silice silanisée avec élution à l'eau. Obtention de cristaux.
m=47g,
m/z : ES+ 155

### b) 3-benzyloxy-2,4-bis(hydroxyméthyl)pyridine :

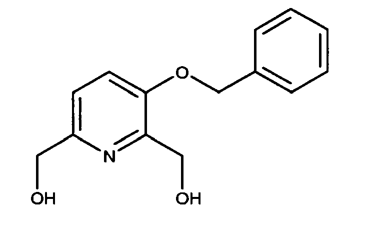

47g (0.3mol) de 3-hydroxy-2,4-bis(hydroxyméthyl)pyridine sont dissous dans 800mL d'acétonitrile. On ajoute 104g de K₂CO₃ (0.753mol) et 55mL de bromure de benzyle (0.45mol). L'ensemble est porté au reflux pendant 12h. Après avoir filtré l'insoluble, la solution est concentrée et le résidu est durci dans l'éther isopropylique.
m=39g,
m /z : ES+ 245

### c) 3-benzyloxy-2,4-bis(bromométhyl)pyridine :

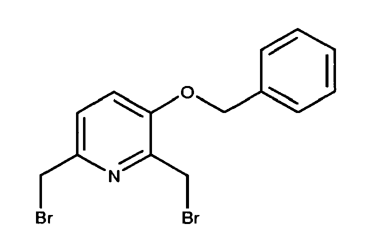

85g (0.347mol) de 3-benzyloxy-2,4-bis(hydroxyméthyl)pyridine sont dissous dans 1000mL d'acétonitrile. La solution est refroidie à 5°C et 419g de CBr₄ et 336g de triphenylphosphine (1.28mol) sont ajoutés en maintenant la température à 5°C. Après 6h de réaction à TA, l'insoluble est filtré et les jus sont concentrés. Le produit est purifié par chromatographie sur silice avec élution au dichlorométhane. On obtient des cristaux blancs.
m/z : ES+ 371

### d) N,N',N"-tris(2-nitro-benzènesulionyl) diéthylènetriamine :

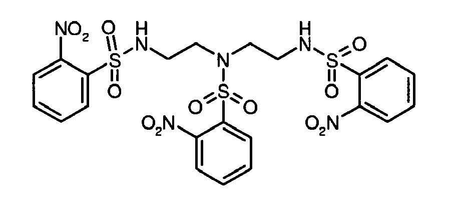

17g (0.165mol) de diéthylènetriamine sont ajoutés à une solution de 84mL de triéthylamine dans 1000mL de dichlorométhane. L'ensemble est refroidi à 0°C. Une solution de 120g (0.544mol) de chlorure de 2-nitro-benzènesulfonyl dissous dans 300mL de dichlorométhane est alors ajoutée goutte à goutte en maintenant la température à 0°C. Laisser revenir doucement la température à TA en 3h. Le mélange est ensuite lavé par de l'eau, la phase organique est séchée puis concentrée. On obtient une poudre blanche.
m/z : ES+ 658

### e) 12-Benzyloxy-3,6,9-tris-(2-nitro-benzènesulfonyl)-3,6,9,15-tétraaza-bicyclo[9.3.1]pentadéca-1(14),11(15),12-triène :

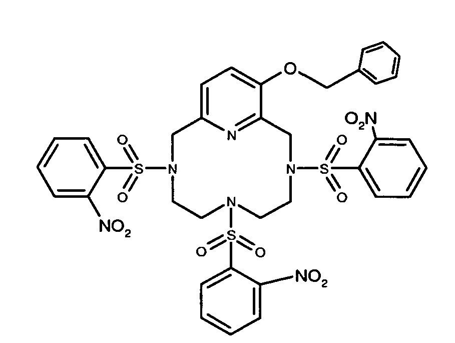

24g (0.058mol) de N,N',N"-Tris(nosyl) diéthylenetriamine sont ajoutés sous une agitation efficace à une solution de 16g de K₂CO₃ en suspension dans 300mL d'acétonitrile. L'ensemble est porté au reflux pendant 2h, puis on introduit 21.5g (0.057mol) de 3-benzyloxy-2,4-bis(bromométhyl)pyridine. Le mélange est maintenu au reflux pendant une nuit. Après filtration du K₂CO₃ et concentration des jus, le produit est purifié par chromatographie sur silice avec élution dichlorométhane/méthanol (9/1).
m=25g,
m/z : ES+ 867

### f) 12-Benzyloxy-3,6,9,15-tétraaza-bicyclo[9.3.1]pentadéca-1(14),11(15),12-triène :

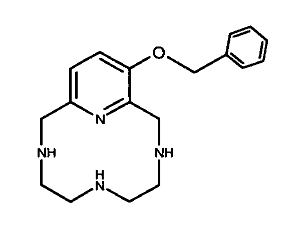

24.2g (0.0279mol) de 12-Benzyloxy-3,6,9-tris-(2-nitro-benzènesulfonyl)-3,6,9,15-tétraaza-bicyclo[9.3.1]pentadéca-1(14),11(15),12-triène sont dissous dans 200mL de DMF, puis on introduit 22g de LiOH et 21mL d'acide thioglycolique.

L'ensemble est laissé une nuit à TA. Après évaporation du DMF, l'huile résiduelle est reprise dans du dichloromethane puis lavée avec de l'eau, séchée et concentrée. On obtient une huile brune.
m=15g,
m/z : ES+ 312

### g) Ester méthylique de l'acide (12-Benzyloxy-6,9-bis-méthoxycarbonylméthyl-3,6,9,15-tétraaza-bicyclo[9.3.1]pentadéca-1(14),11(15),12-trièn-3-yl)-acétique

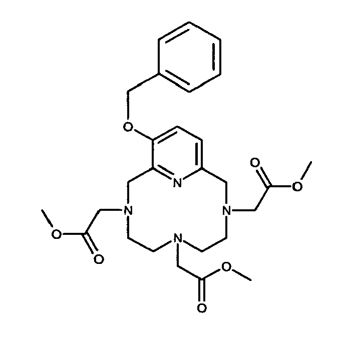

15g (0.0279mol) de 12-Benzyloxy-3,6,9,15-tétraaza-bicyclo[9.3.1]pentadéca-1(14),11(15),12-triène sont ajoutés à une solution de 35g de K₂CO₃ et 25mL de bromoacétate de methyle dans 370mL d'acétonitrile. L'ensemble est porté au reflux pendant 18h. Après filtration, la solution est concentrée, reprise dans 200mL d'HCl 1N puis lavée par de l'éther. On neutralise. On extrait au dichloromethane et on concentre. On obtient une huile jaune.
m=9g,
m/z : ES+ 522

### h) Acide(12-Benzyloxy-6,9-bis-carboxyméthyl-3,6,9,15-tétraaza-bicyclo[9.3.1]pentadéca-1(14),11(15),12-trièn-3-yl)-acétique:

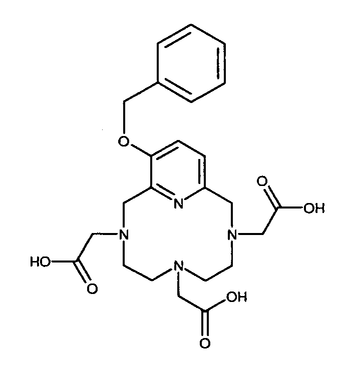

9g (0.0170mol) de l'ester méthylique de l'acide (12-Benzyloxy-6,9-bis-méthoxycarbonylméthyl-3,6,9,15-tétraaza-bicyclo[9.3.1]pentadéca-1(14),11(15),12-trien-3-yl)-acétique sont ajoutés à une solution de 50mL de méthanol et 50mL de NaOH 5N. L'ensemble est porté au reflux 6h. Après concentration et neutralisation par passage sur résine IRC50, le produit est durci dans l'isopropanol. On obtient des cristaux blancs.
m=8g,
m/z : ES+ 486

### i) Acide (6,9-Bis-carboxyméthyl-12-hydroxy-3,6,9,15-tétraaza-bicyclo[9.3.1]pentadéca-1(14),11(15),12-trièn-3-yl)-acétique :

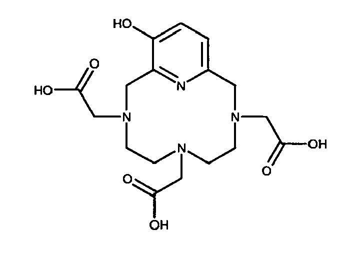

8g (0.0170mol) de l'acide (12-Benzyloxy-6,9-bis-carboxyméthyl-3,6,9,15-tétraaza-bicyclo[9.3.1]pentadéca-1(14),11(15),12-trièn-3-yl)-acétique sont ajoutés à 100mL d'HCl 10N. On laisse réagir une nuit à 40°C. On concentre. Lle produit est cristallisé dans l'acétone.
m=6g,
m/z : ES+ 396

### j) Complexe de gadolinium du (6,9-Bis-carboxyméthyl-12-hydroxy-3,6,9,15-tétraaza-bicyclo[9.3.1]pentadéca-1(14),11(15),12-trien-3-yl)-acétique :

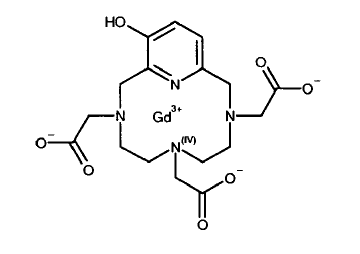

5g (0.0126mol) de l'acide (6,9-Bis-carboxyméthyl-12-hydroxy-3,6,9,15-tétraaza-bicyclo[9.3.1]pentadéca-1(14),11(15),12-trièn-3-yl)-acétique sont dissous dans 80mL d'eau. Le pH est ajusté à 6 par NaOH 2N et la température portée à 60°C. On ajoute 2.32g (0.00631mol) de Gd₂O₃. L'ensemble est agité à 60°C pendant 6h en maintenant le pH à 6. Après filtration d'un trouble, la solution est concentrée, puis le produit est durci dans l'éthanol. On obtient des cristaux blancs.
m=6.3g.
m/z : ES+ 550

### k) Complexe de gadolinium du (12-Carboxyméthoxy-6,9-bis-carboxyméthyl-3,6,9,15-tétraaza-bicyclo[9.3.1]pentadéca-1(14),11(15),12-trien-3-yl)-acétique :

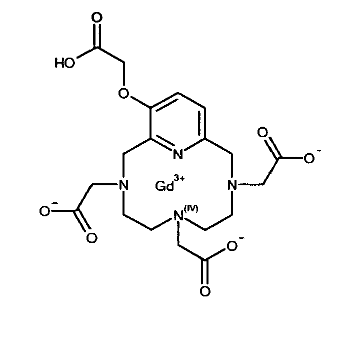

550mg (0.001mol) de complexe de gadolinium de l'acide (6,9-Bis-carboxyméthyl-12-hydroxy-3,6,9,15-tétraaza-bicyclo[9.3.1 ]pentadeca-1(14),11(15),12-trièn-3-yl)-acetique sont ajoutés à une solution de 20mL d'eau contenant 100mg (0.005mol) de NaOH. On ajoute 180mg (0.0015mol) d'acide bromoacétique. La solution est portée à 50°C et laissée une nuit sous agitation. Après concentration et reprise dans l'éthanol, on obtient des cristaux blancs.
m=600mg,
m/z : ES+ 608

### EXEMPLE 5

a) Complexe de gadolinium de l'ester de l'acide Octadécanoique 3-({2-[4-(3,9-bis-carboxyméthyl-3,6,9,15-tétraaza-bicyclo[9.3.1]pentadéca-1(14),11(15), 12-trien-6-yl)-4-carboxy-butyrylamino]-éthoxy}-hydroxy-phosphoryloxy)- 2-octadécanoyloxy-propylique 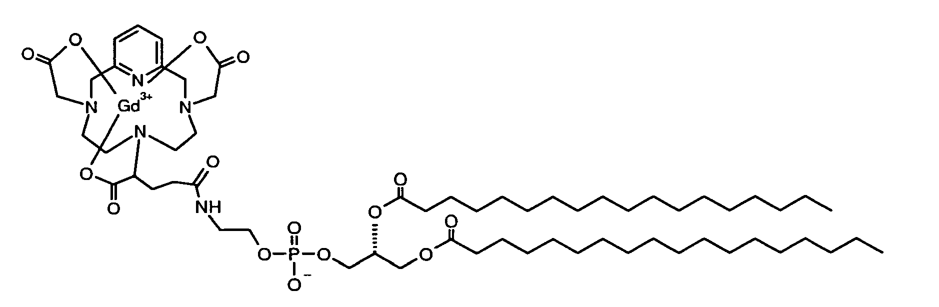 200 mg du composé obtenu à l'étape c) de l'exemple 3 sont dissous dans 10 ml de Diméthylformamide. A cette solution sont ajoutés 204 mg de N,N'-dicyclohexylcarbodiimide et 40mg de N-hydroxysuccunimide. Le mélange est agité 1h à température ambiante et une solution de 250 mg de 1,2-Distearoyl-sn-Glycero-3-Phosphoethanolamine (DSPE, AVANTI^{®} Polar Lipids, Inc.) dans 5 ml de pyridine est ajoutée. Le milieu réactionnel est agité 20h à température ambiante puis précipité dans 50 ml d'éthanol .Le produit est ensuite purifié sur gel de silice. m= 190mg.
   m/z : ES- 1335

### EXEMPLE 6

a) Complexe de gadolinium de l'acide 2-(3,9-Bis-carboxyméthyl-3,6,9,15-tétraaza-bicyclo[9.3.1]pentadéca-1(14),11(15),12-trien-6-yl)-4-(4-octadéc-9-ènoylamino-phényl)-butyrique 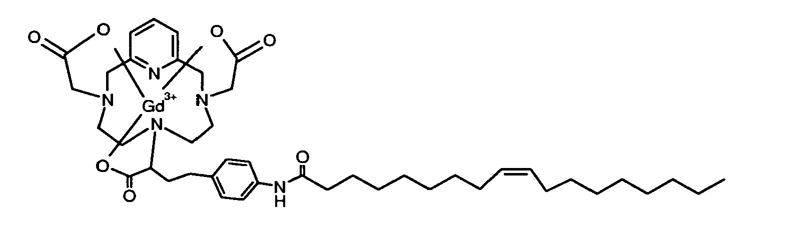 500mg du composé obtenu à l'étape j) de l'exemple 1 sont dissous dans 30 ml de DMSO anhydre. 230 mg de triéthylamine sont ajoutés puis 400mg de chlorure de l'acide Oléique (ALDRICH^{®} ). Le mélange est agité 6h à température ambiante et précipité dans l'éthanol. Le produit est ensuite purifié sur gel de silice.m=300 mg.
   m/z : ES- 917

### EXEMPLE 7

a) Complexe de gadolinium de l'acide 2-(6,9-Bis-carboxyméthyl-3,6,9,15-tétraaza-bicyclo[9.3.1]pentadéca-1(14),11(15),12-trièn-3-yl)-4-(4-hexadécanoylamino-phényl)-butyrique 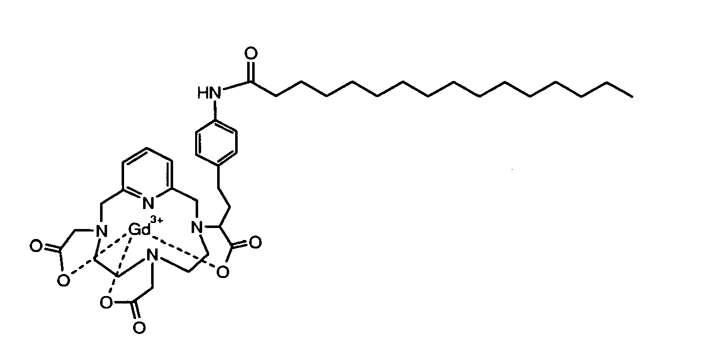 100 mg de composé obtenu à l'étape e) de l'exemple 2 sont dissous dans 20ml de DMSO anhydre. 50mg de triéthylamine puis 63 mg du chlorure de l'acide Palmitique (ALDRICH^{®}) sont ajoutés et le mélange est agité 6h à température ambiante. Après précipitation dans l'éthanol et purification sur gel de silice on obtient 56 mg de produit.
   m/z : ES- 891

### EXEMPLE 8

a) Complexe de gadolinium de l'ester 2-héxadécanoyloxy-3-(hydroxy-{2-[2-(3,6,9-tris-carboxyméthyl-3,6,9,15-tétraaza-bicyclo[9.3.1]pentadéca-1(15),11,13-trien-12-yloxy)-acétylamino]-éthoxy}-phosphoryloxy)-propylique de l'acide Hexadécanoique 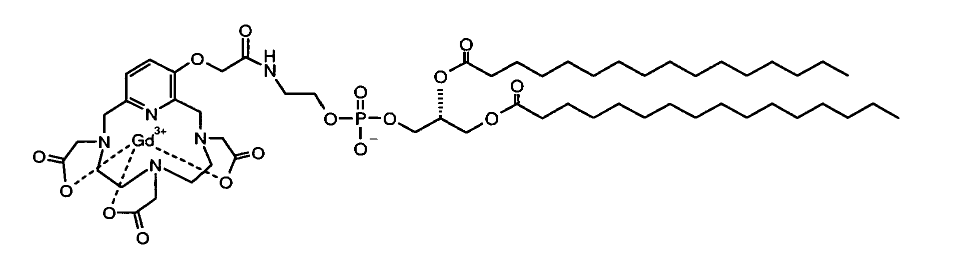 200mg du composé obtenu à l'étape k) de l'exemple 4 sont dissous dans 20 ml de 1-methyl-2-pyrolidinone et 500 mg de bromotripyrrolidinophosphonium hexafluorophosphate (PyBroP^{®} ALDRICH^{®}) puis 230 mg de 1,2-Dipalmitoyl-sn-Glycero-3-Phosphoéthanolamine (DPPE, AVANTI^{®} Polar Lipids, Inc) sont ajoutés. Le milieu réactionnel est agité 24 h à température ambiante puis précipité dans 50 ml d'éthanol. On obtient après purification sur gel de silice 126 mg de produit.
   m/z : ES- 1280

### EXEMPLE 9

a) Complexe de gadolinium de l'acide 2-(6,9-Bis-carboxyméthyl-3,6,9,15-tétraaza-bicyclo[9.3.1]pentadéca-1(14),11(15),12-trien-3-yl)-4-(4-isothiocyanato-phényl)-butyrique 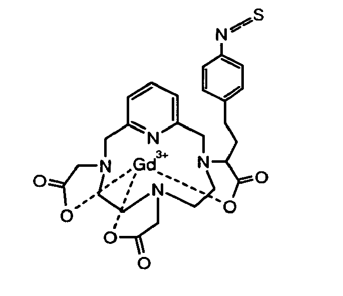 2 g du produit issu de l'étape e) de l'exemple 2 sont dissous dans un mélange de 24 ml d'eau et 16 ml de CHCl₃. 0.5 ml (6.5 10⁻³ m)de thiophosgène est additionné goutte à goutte et l'ensemble est agité 1 heure 30. Le milieu réactionnel est décanter et la phase aqueuse est évaporée sous vide. Le résidu est repris dans de l'éther éthylique et agité une nuit à température ambiante.
   Le précipité est filtré et séché sous vide. m= 2,1g
   m/z : ES- 695

### EXEMPLE 10

a) Complexe de gadolinium de l'acide 2-(6,9-Bis-carboxyméthyl-3,6,9,15-tétraaza-bicyclo[9.3.1] pentadéca-1(14),11(15),12-trien-3-yl)-4-[4-(3-octadéc-9-enyl-thioureido)-phényl] -butyrique

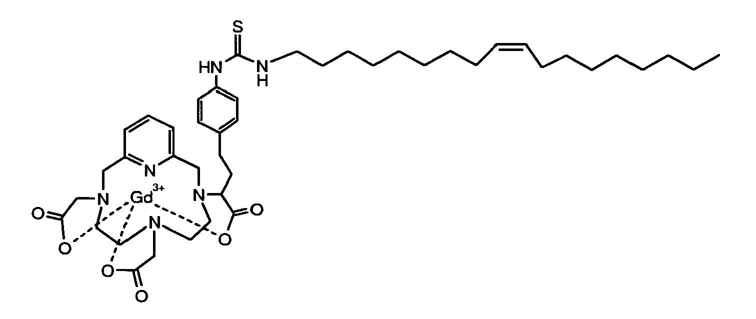

Le composé obtenu à l'étape a) de l'exemple 9 (1g) est dissous à température ambiante dans 20 ml de DMSO. L'Oléylamine ( 550 mg) est ensuite ajoutée et le milieu réactionnel agité pendant 48h avant d'être précipité dans 200ml d'éther éthylique. Le précipité est lavé avec de l'ether éthylique puis de l'éthanol. Après purification sur gel de silice on obtient m= 650mg de produit.
m/z : ES- 962

### EXEMPLE 11

### a) Ester méthylique de l'acide (13-Bromo-6,9-bis-éthoxycarbonylméthyl-3,6,9,15-tétraaza-bicyclo[9.3.1]pentadéca-1(14),11(15),12-trièn-3-yl)-acétique

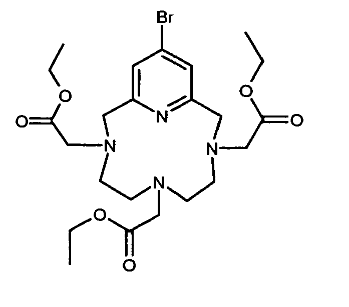

22 g de 13-bromo-3,6,9,15-tétraazabicyclo[9.3.1.]pentadéca-1(15),11,13-triène obtenus selon
J. Heterocyclic Chem. 27, 1990, pages 167-169, sont introduits dans 440 ml de CH₃CN en présence de 48 g de K₂CO₃ calciné et le mélange est maintenu à 80° C pendant 1 h avant l'addition d'une solution de 50 g de Bromoacétate d'éthyle dans 100 ml de CH₃CN ; le milieu réactionnel est alors agité 20 h à 80° C puis refroidi à température ambiante, filtré et le solvant est évaporé. Le résidu est repris par 500 ml d'une solution aqueuse de HCl 1N en présence d'un volume d'éther diéthylique. Après séparation de la phase organique, la phase aqueuse est neutralisée par NaHCO₃ puis extraite par CH₂Cl₂. Après lavage à l'eau puis séchage sur sulfate de magnésium, la phase organique est concentrée et le résidu est purifié sur colonne de silice (Merck^{®} 500 g, d = 10 cm) en éluant par CH₃COOC₂H₅.
m = 20.9 g ;
m/z : ES+ 544,6

### b) Ester éthylique de l'acide [13-(3-tert-Butoxycarbonylamino-propènyl)-6,9-bis-éthoxycarbonylméthyl-3,6,9,15-tétraaza-bicyclo[9.3.1]pentadéca-1(14),11(15),12-trièn-3-yl] -acétique

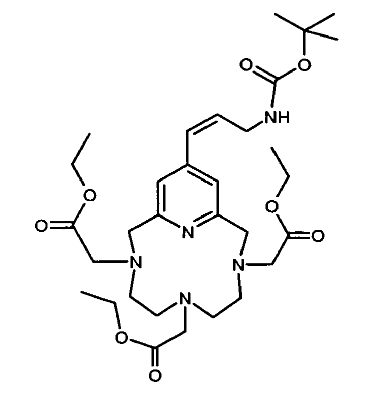

On ajoute 6 g de 3-(tert-butyloxycarbonylamino)-propène, 10 ml de triéthylamine, puis 800mg de triphénylphosphine et enfin 400 mg d'acétate de palladium à une solution de 5g du composé obtenu à l'étape a) dissous dans 100 ml de toluène. Après chauffage à 80° C pendant une nuit sous atmosphère inerte, le milieu est évaporé et le résidu est repris par une solution aqueuse d'acide chlorhydrique (pH = 1). La phase aqueuse est lavée avec 1 volume d'éther diéthylique puis de toluène avant d'être amenée à pH 6 par addition de NaOH (1N).

Après extraction de la solution aqueuse par CH₂Cl₂, la phase organique séchée sur sulfate de magnésium est évaporée. On obtient une huile marron qui est chromatographiée sur gel de silice.
m = 2,8 g
m/z : ES+ 621

### c) Ester éthylique de l'acide [13-(3-tert-Butoxycarbonylamino-propyl)-6,9-bis-éthoxycarbonylméthyl-3,6,9,15-tétraaza-bicyclo[9.3.1]pentadéca-1(14),11(15),12-trièn-3-yl]-acétique

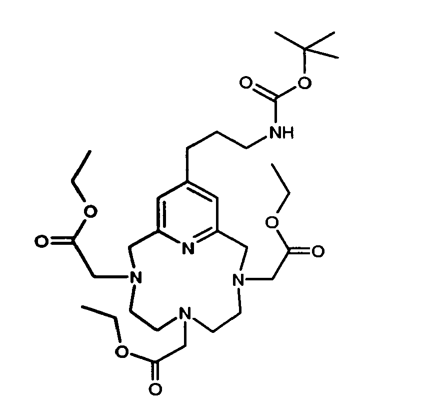

A 2 g du composé obtenu à l'étape b) dissous dans 80 ml de CH₃OH, on ajoute 200mg de catalyseur charbon palladié à 10% puis le milieu réactionnel est agité durant 2 h 30 à 20° C sous 4.10⁵ Pa d'hydrogène. Après filtration sur Clarcel^{®}, le solvant est évaporé et on obtient 1,8 g d'huile après chromatographie sur gel de silice.
m/z : ES+ 623

### d) Acide [13-(3-tert-Butoxycarbonylamino-propyl)-6,9-bis-carboxyméthyl-3,6,9,15-tétraaza-bicyclo[9.3.1]pentadéca-1(14),11(15),12-trien-3-yl]-acétique

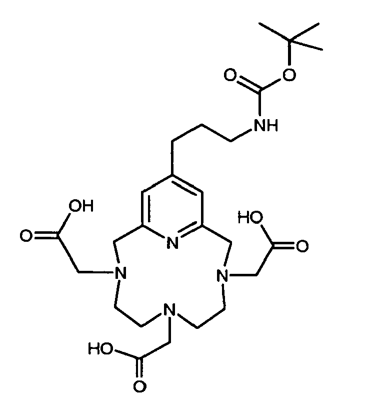

1g du composé obtenu à l'étape c) dissous dans 20 ml d'une solution aqueuse de NaOH 5N et 20 ml de CH₃OH sont chauffés à 70°C pendans 18 h. Après concentration du milieu réactionnel, le résidu est repris dans l'eau et la solution, amenée à pH 5,5-6 par quelques gouttes d'acide acétique, est concentrée avant d'être purifiée par chromatographie sur une colonne (d = 15 cm) contenant 50 g de silice silanisée (Merck^{®} 0,063 - 0,200 µm) en éluant à l'eau. Après concentration à sec on obtient 480mg de cristaux blancs.
m/z : ES- 536,5

### e) Complexe de gadolinium de l'acide [13-(3-tert-Butoxycarbonylamino-propyl)-6,9-bis-carboxyméthyl-3,6,9,15-tétraaza-bicyclo[9.3.1]pentadéca-1 (14),11(15),12-trièn-3-yl]-acétique

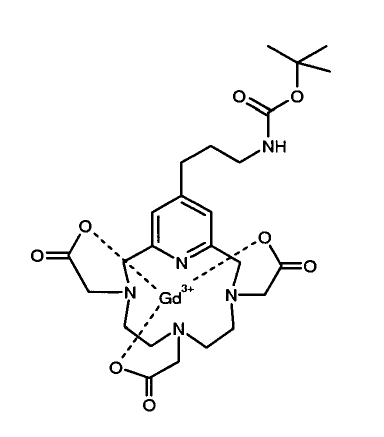

300mg du composé obtenu à l'étape d) sont dissous dans 10 ml d'eau puis on ajoute en une fois 100mg de Gd₂O₃ et l'ensemble est chauffé à 60° C durant 3 h 45 min en maintenant le pH entre 5,5 et 6 par addition d'une solution aqueuse de NaOH 1N. Après filtration, le milieu réactionnel est évaporé et le résidu est cristallisé dans l'éthanol. Après traitement par une résine Chelex^{®} 100 (Bio-Rad) on obtient 320mg de cristaux blancs.
m/z : ES- 691

### f) Complexe de gadolinium de l'acide [13-(3-Amino-propyl)-6,9-bis-carboxyméthyl-3,6,9,15-tétraaza-bicyclo[9.3.1]pentadéca-1(14),11(15),12-trièn-3-yl]-acétique

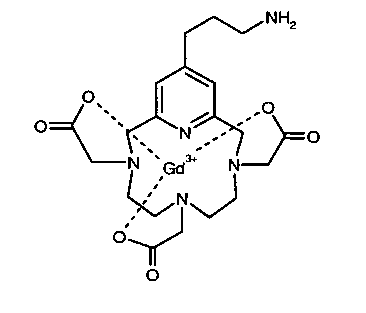

On maintient 3 h sous agitation une solution de 300mg du complexe obtenu à l'étape e) dans 18ml de CF₃COOH à 25° C avant d'éliminer le liquide sous pression réduite.

Le résidu est repris dans l'éther diéthylique et la suspension filtrée. Après élimination du solvant, le résidu est introduit par portions dans une suspension d'au moins 1 ml de résine anionique faible (OH-) dans 5 ml d'eau ; en fin d'addition le pH, stable, doit être de 8 à 8,5.

La résine est alors séparée par filtration, le solvant éliminé et le résidu précipité par addition d'éther éthylique.m= 200mg.
m/z : ES+ 593

### EXEMPLE 12

### a) Complexe de gadolinium de l'acide {6,9-Bis-carboxyméthyl-13-[3-(2-éthoxy-3,4-dioxo-cyclobut-1-ènylamino) -propyl]-3,6,9,15-tétraaza-bicyclo[9.3.1]pentadéca-1(14),11(15),12-trièn-3-yl}-acétique

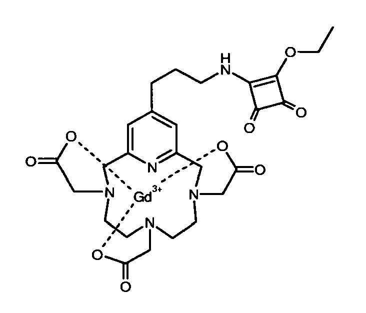

1 g de composé obtenu à l'étape f) de l'exemple 11 sont séchés au toluène, puis mis en suspension dans 20ml de DMSO anhydre sous couverture d'argon.0,4 ml de Et₃N séchée sur tamis (1.7 éq) et 720 mg de diéthylsquarate (Aldrich, 2.5 éq.).sont alors ajoutés. Le mélange est agité à température ambiante sous couverture d'argon pendant 1 heure. Le milieu est précipité dans 120 ml d'éther. L'huile jaunâtre est lavée à l'éther éthylique. Le solide obtenu est filtré puis lavé au Dichlorométhane.
On obtient après filtration 700mg d'un solide blanc.
m/z : ES- 718

### EXEMPLE 13

### a) Ester benzylique de l'acide 2-(3,9-Bis-éthoxycarbonylméthyl-3,6,9,15-tétraaza-bicyclo[9.3.1]pentadéca-1(14),11(15),12-trièn-6-yl)-5-(1,3-dioxo-1,3-dihydro-isoindol-2-yl)-pentanoique

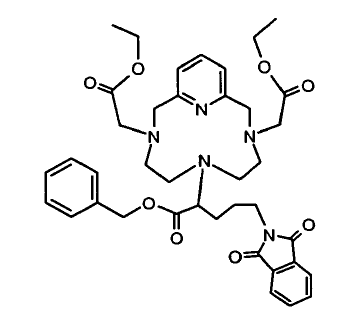

Au départ du composé obtenu à l'étape f) de l'exemple 1 (1g) et de l'ester benzylique de l'acide 2-Bromo-5-(1,3-dioxo-1,3-dihydro-isoindol-2-yl)-pentanoique (1,5g) selon le mode opératoire décrit à l'étape g) de l'exemple 1. Après purification sur gel de silice, 700mg de produit sont obtenus.
m/z : ES+ 715

### b) Acide 5-Amino-2-(3,9-bis-carboxyméthyl-3, 6,9,15-tétraaza-bicyclo[9.3.1]pentadéca-1(14),11(15),12-trièn-6-yl)-pentanoique

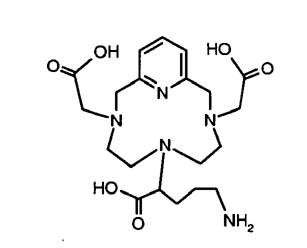

500 mg de produit sont obtenus selon le mode opératoire de l'étape h) de l'exemple 1 au départ du composé obtenu à l'étape a) ( 1,6g).
m/z : ES- 436,5

### c) Complexe de gadolinium de l'acide 5-Amino-2-(3,9-bis-carboxyméthyl-3, 6,9,15-tétraaza-bicyclo[9.3.1]pentadéca-1(14),11(15),12-trièn-6-yl)-pentanoique

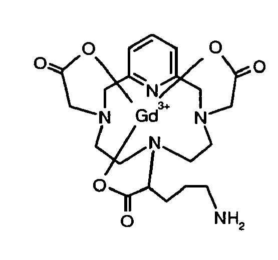

Selon le mode opératoire de l'étape i) de l'exemple 1 au départ de 500mg du composé obtenu à l'étape b). m= 620mg
m/z : ES+ 593

### EXEMPLE 14

**a) Complexe de gadolinium de l'acide 2-(3,9-Bis-carboxyméthyl-3,6,9,15-tétraaza-bicyclo[9.3.1]pentadéca-1(14),11(15),12-trièn-6-yl)-5-(2-éthoxy-3,4-dioxo-cyclobut-1-énylamino)-pentanoique**

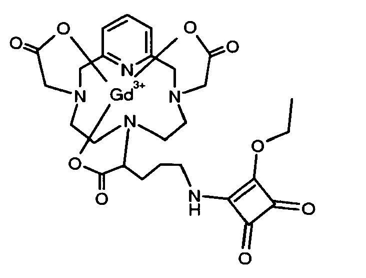

Selon le mode opératoire de l'étape a) de l'exemple 12 au départ de 500mg du composé obtenu à l'étape c) de l'exemple 13. m= 410mg
m/z : ES+ 717

### EXEMPLE 15

### a) Ester diéthylique de l'acide 2-Bromo-hexanedioique

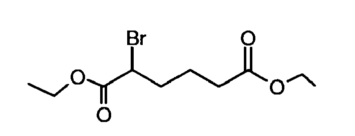

75g du monoester éthylique de l'acide Hexanedioique sont ajoutés à 123mL de SOCl₂ puis portés 3h au reflux. 26.4mL de Br₂ sont ajoutés goutte à goutte en maintenant le reflux. On laisse la nuit à température ambiante. La solution est versée lentement à 0°C dans 225mL d'éthanol. Après addition, le milieu est laissé 2h à 0°C puis versé lentement dans 600mL d'eau glacée. La phase aqueuse est extraite à l'éther éthylique, et la phase éthérée est lavée avec une solution à 10% de NaHCO₃ puis à l'eau. Après évaporation de l'éther, l'huile obtenue est distillée.
m=80g
m/z : ES+ 281

### b) Ester diéthylique de l'acide 2-(3,9-Bis-éthoxycarbonylméthyl-3,6,9,15-tétraaza-bicyclo[9.3.1]pentadéca-1(14),11(15),12-trièn-6-yl)-hexanedioique

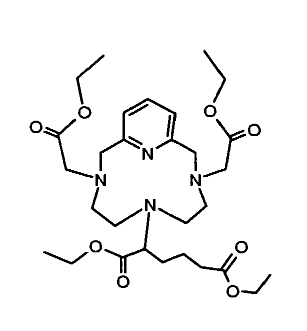

En appliquant le même mode opératoire que pour l'étape g) de l'exemple 1 on obtient 750mg de produit au départ de 1 g de composé obtenu à l'étape f) de l'exemple 1.et 1.1g de produit obtenu à l'étape a), .après chromatographie sur colonne silice (Merck^{®} 40-60 µm) en éluant avec un mélange CH₂Cl₂/acétone 70/30 v/v
m/z : ES+ 580

### c) Acide 2-(3,9-Bis-carboxymethyl-3,6,9,15-tétraaza-bicyclo[9.3.1]pentadéca-1(14),11(15),12-trièn-6-yl)-hexanedioique

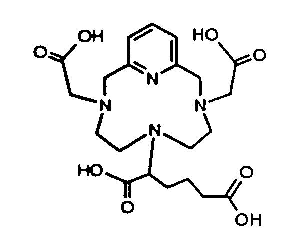

En appliquant le même mode opératoire que pour l'étape h) de l'exemple 1, on obtient 220mg de produit à partir de 500 mg du composé obtenu à l'étape b).
m/z : ES- 465

### d) Complexe de gadolinium de l'acide 2-(3,9-Bis-carboxyméthyl-3,6,9,15-tétraaza-bicyclo[9.3.1]pentadéca-1(14),11(15),12-trièn-6-yl)-hexanedioique

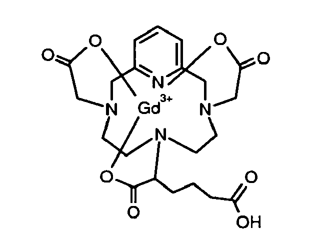

En appliquant le même mode opératoire que pour l'étape i) de l'exemple 1, on obtient 180 mg de produit à partir de 200mg du composé obtenu à l'étape c).
m/z : ES- 620

### EXEMPLE 16

### a) Ester diméthylique de l'acide 2-(3,9-Bis-éthoxycarbonylméthyl-3,6,9,15-tétraaza-bicyclo[9.3.1]pentadéca-1(14),11(15),12-trièn-6-yl)-but-2-énedioique

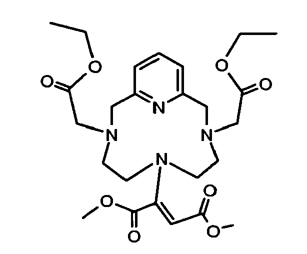

A une solution de 500mg de composé obtenu à l'étape f) de l'exemple 1 dans 20 ml d'acétonitrile, sont ajoutés 380mg d'acéthylènedicarboxylate de diméthyle et le mélange est porté à 55°c durant 1H. Après refroidissement, le solvant est évaporé sous pression réduite et l'huile résiduelle est purifiée sur colonne silice (Merck^{®} 40-60 µm) en éluant avec un mélange Acétate d'éthyle/Heptane. m= 420mg
m/z : ES+ 521.6

### b) Ester diméthylique de l'acide 2-(3,9-Bis-éthoxycarbonylméthyl-3,6,9,15-tétraaza-bicyclo[9.3.1]pentadéca-1(14),11(15),12-trièn-6-yl)-succinique

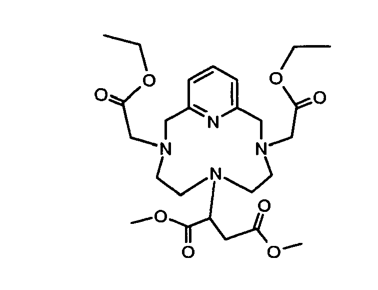

A une solution composée de 400mg du produit obtenu à l'étape a), de 10ml d'écétonitrile et de 2ml d'acide acétique sont ajouté 300mg de NaBH₃CN. Le milieu est agité 12h à température ambiante. Le solvant est évaporé sous pression réduite , l'huile résiduelle est dissoute dans le dichlorométhane et la solution est lavée à l'eau bicarbonatée jusqu'à neutralité des eaux de lavage. Après séchage et évaporation de la solution chlorométhylènique, 380mg de produit sont obtenus.
m/z : ES+ 523,7

### c) Acide 2-(3,9-Bis-carboxyméthyl-3,6,9,15-tétraaza-bicyclo[9.3.1]pentadéca-1(14),11(15),12-trièn-6-yl)-succinique

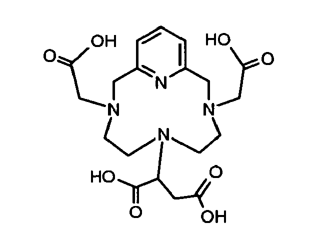

Selon le mode opératoire de l'étape h) de l'exemple 1 au départ de 300mg de produit obtenu à l'étape b). m= 180mg
m/z : ES- 437

### d) Complexe de gadolinium de l'acide 2-(3,9-Bis-carboxyméthyl-3,6,9,15-tétraaza-bicyclo[9.3.1]pentadéca-1(14),11(15),12-trièn-6-yl)-succinique

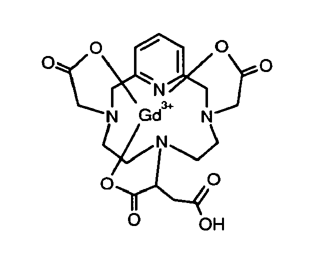

Selon le mode opératoire de l'étape i) de l'exemple 1 au départ de 150mg de composé obtenu à l'étape c). m= 170mg
m/z : ES- 591,5

### EXEMPLE 17

### a) Octadecylamine

2g d' oléylamine dans 120ml d'éthanol sont hydrogénés à 30°c sous 8 bars d'hydrogène en présence de Palladium sur charbon. Après 4h de réaction et évaporation du solvant, 1.6g de produit sous forme d'une poudre blanche sont obtenus.
m/z : ES+ 270,7

### b) acide 2-(3,9-Bis-carboxyméthyl-3,6,9,15-tétraaza-bicyclo[9.3.1]pentadéca-1(14),11(15),12-trièn-6-yl)-N-octadecyl-succinamique

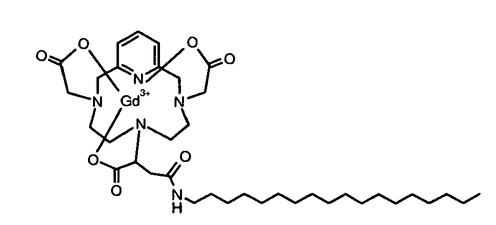

A une solution de 100mg du composé préparé à l'étape d) de l'exemple 16 dans 10ml de DMF sont ajoutés 50mg de l'amine préparée à l'étape a), 70mg de DCC (dicyclohexylcarbodiimide) et 50µl de triéthylamine. Le milieu est agité 18h à température ambiante puis précipité dans l'éthanol. Le précipité est filtré, rincé à l'éther éthylique et séché sous pression réduite. Après purification sur gel de silice, on obtient m=70mg de produit.
m/z : ES- 843

### EXEMPLE 18

### a) Complexe de gadolinium de l'acide 2-(3,9-Bis-carboxyméthyl-3,6,9,15-tétraaza-bicyclo[9.3.1]pentadéca-1(14),11(15),12-trièn-6-yl)-5-(2-octadécylamino-3,4-dioxo-cyclobut-1-énylamino)-pentanoique

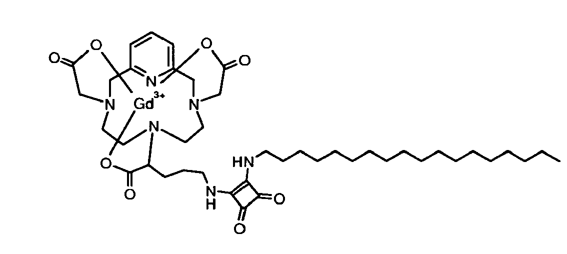

150mg de l'amine préparée à l'étape a) de l'exemple 17 et 400 mg du composé préparé à l'étape a) de l'exemple 14 sont mis en suspension dans 4ml de DMSO et trois équivalents de triéthylamine sont ajouté. Le mélange est agité à 50°c 24h. le milieu réactionnel est précipité dans 40ml d'éthanol et le solide obtenu est lavé à l'éther éthylique et séché sous pression réduite.m= 460mg
m/z : ES+ 940

### EXEMPLE 19

### b) Complexe de gadolinium de l'ester 3-[(2-{3,4-dioxo-2-[3-(3,6,9-tris-carboxyméthyl-3,6, 9,15-tétraaza-bicyclo[9.3.1]pentadéca-1(14),11(15),12-trièn-13-yl)-propylamino]-cyclobut-1-énylamino}-éthoxy)-hydroxy-phosphoryloxy]-2-octadécanoyloxy-propylique de l'acide Octadécanoique

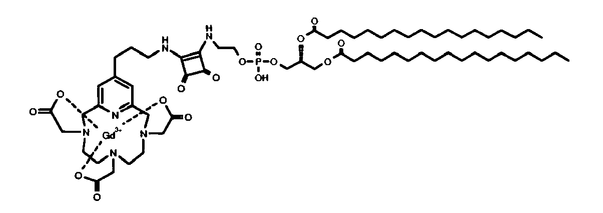

Selon le mode opératoire de l'étape a) de l'exemple 18, au départ de 500mg du composé préparé à l'étape a) de l'exemple 12 et 520mg de DSPE. m= 350 mg
m /z : ES- 1417

### EXEMPLE 20

### a) Complexe de gadolinium de l'acide 2-(3,9-Bis-carboxymethyl-3,6,9,15-tetraaza-bicyclo[9.3.1]pentadeca-1(14),11(15),12-trien-6-yl)-5-hexadécanoylamino-pentanoique

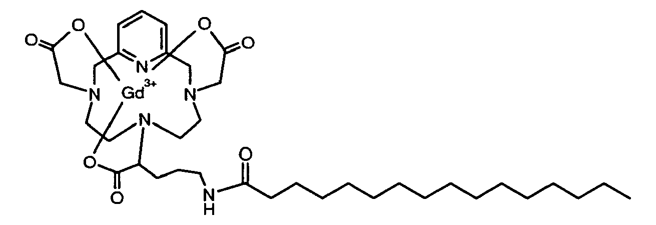

Selon le mode opératoire de l'étape a) de l'exemple 6 au départ du composé obtenu à l'étape c) de l'exemple 13 (300mg) et 150mg du chlorure de l'acide palmitique.m= 230mg
m /z : ES- 829

### EXEMPLE 21

### a) Complexe de gadolinium de l'ester 3-({2-[5-(3,9-bis-carboxyméthyl-3,6,9,15-tétraaza-bicyclo[9.3.1]pentadéca-1(14),11(15), 12-trièn-6-yl)-5-carboxy-pentanoylamino]-éthoxy}-hydroxy-phosphoryloxy)-2-héxadecanoyloxy-propylique de l'acide Hexadécanoique

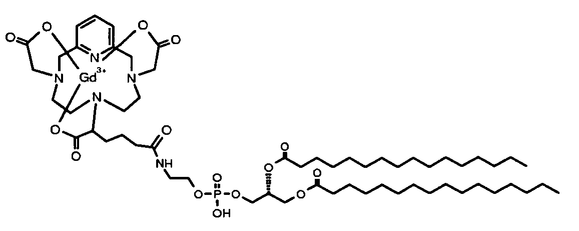

Selon le mode opératoire de l'étape a) de l'exemple 8, au départ de 100mg du composé préparé à l'étape d) de l'exemple 15 et 120mg de DPPE. m= 80 mg
m/z:ES- 1293

### EXEMPLE 22

### a) Acide [6,9-Bis-carboxyméthyl-13-(3-hexadécanoylamino-propyl)-3,6,9,15-tétraaza-bicyclo[9.3.1]pentadéca-1(14),11 (15),12-trièn-3-yl]-acétique

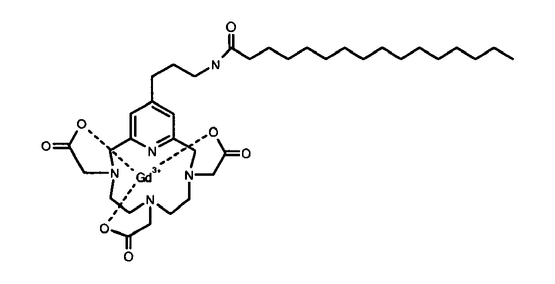

Selon le mode opératoire de l'étape a) de l'exemple 6 au départ du composé obtenu à l'étape) de l'exemple 11 (150mg) et 100mg du chlorure de l'acide palmitique m= 110mg
m /z : ES- 829.

## Revendications

1. Composé de formule (I) 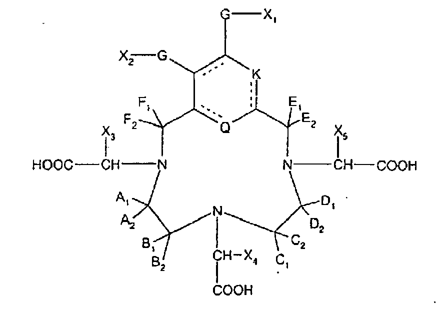 dans laquelle :
1) --- représente une simple ou double liaison ;
2) Q représente un atome d'azote ou un radical NH ;
3) X₁, X₂, X₃, X₄ et X₅, identiques ou différents, représentent indépendamment ;
3.1) un atome d'hydrogène ;
3.2) un groupe -(CH₂)ₐ-CONR₁R₂, -(CH₂)ₐ-NR₁COR₂ ou 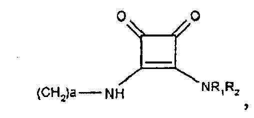 avec ;
chaque R₁, R₂ représente indépendamment un atome H ; une chaîne alkyle ou alcényle en C₇-C₃₀, substituée ou non substituée, linéaire ou ramifiée ou cyclique, éventuellement interrompue par O, NH, NR₃ ou S, où R₃ est un alkyle en C₁-C₃, ou un groupe 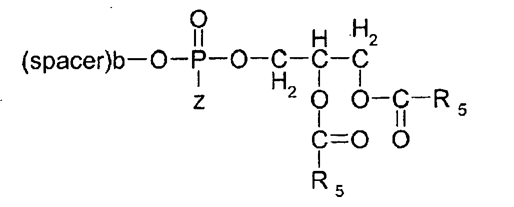 avec b = 0, 1 ou 2 , Z représente O⁻ ou OH, R₅ représente un groupe saturé ou insaturé d'au moins 6 atomes de carbone avantageusement en C₆ à C₃₀, éventuellement substitué, et spacer représente un groupe CH₂CH₂ ou polyalkylèneglycol ;
3.3) un groupe 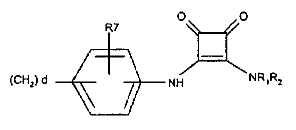 ou 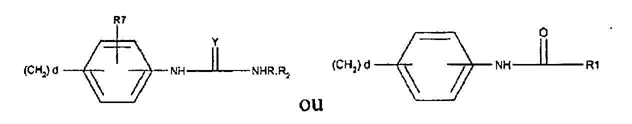 avec :
d compris entre 0 et 3 ;
Y choisi parmi O et S ;
R₇ choisi parmi H, OH, CH₃, OCH₃;
R₁ et R₂ sont tels que définis ci-dessus
à la condition que les X₁, X₂, X₃, X₄ et X₅ ne soient pas tous H et qu'au moins un des X₁, X₂, X₃, X₄, X₅ soit un groupe lipophile choisi parmi :
• un groupe -(CH₂)ₐ-CONR₁R₂ -(CH₂)ₐ-NR₁COR₂ ou 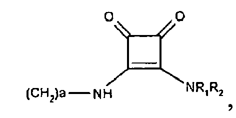 avec ;
chaque R₁, R₂ représentent indépendamment une chaîne alkyle ou alcényle en C₇ à C₃₀ linéaire ou ramifié ou cyclique éventuellement interrompue par un ou deux atomes ou groupes O, S, NH ou NR₃ où R₃ est un alkyle en C₁-C_{3;} ou un groupe 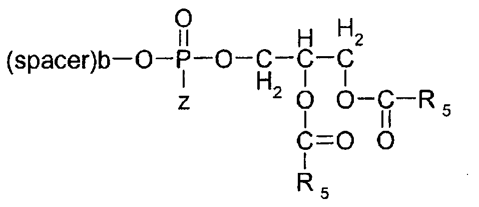 avec b = 0, 1 ou 2, Z représente O⁻ ou OH, R₅ représente un groupe saturé ou insaturé d'au moins 6 atomes de carbone avantageusement en C₆ à C₃₀, éventuellement substitué, et spacer représente un groupe CH₂CH₂ ou polyalkylèneglycol ;
• un groupe 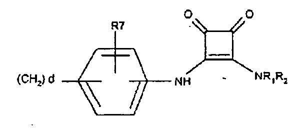
ou 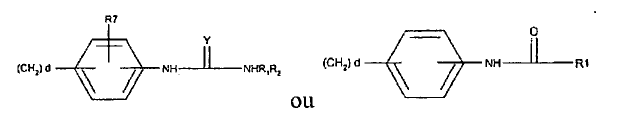 avec :
d compris entre 0 et 3 ;
Y choisi parmi O et S ;
R₇ choisi parmi H, OH, CH₃, OCH₃ ;
chaque R₁, R₂ représentent indépendamment une chaîne alkyle ou alcényle en C₇ à C₃₀ linéaire ou ramifié ou cyclique éventuellement interrompue par un ou deux atomes ou groupes O, S, NH, ou NR₃ où R₃ est un alkyle en C₁-C_{3 ;} ou un groupe 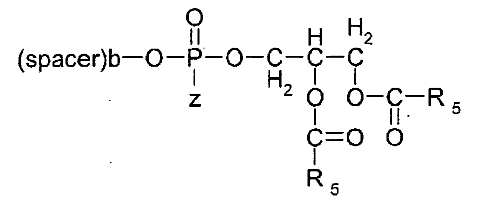 avec b = 0, 1 ou 2, Z représente O⁻ ou OH, R₅ représente un groupe saturé ou insaturé d'au moins 6 atomes de carbone avantageusement en C₆ à C₃₀, éventuellement substitué, et spacer représente un groupe
CH₂CH₂ ou polyalkylèneglycol ;
4) A₁, A₂, B₁, B₂, C₁, C₂, D₁, D₂, E₁, E₂, F₁, F₂, représentent indépendamment les uns des autres un groupe H ou CH₃ ou cyclohexyle ;
5) K représente C ou N ou CH ou NH ou N⁺R₄ avec R₄ un groupe en C₁-C₆ choisi parmi alkyle, benzyle ou benzyle substitué ;
6) G représente rien ou O ou NHR₄, R₄ est tel que défini ci-dessus et ses sels pharmaceutiquement acceptables.

2. Composé de formule (I) 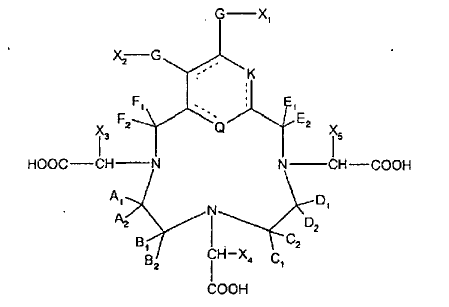 dans laquelle :
1) --- représente une simple ou double liaison
2) Q représente un atome d'azote ou un radical NH
3) X₁ ou X₂ identiques ou différents, représentent indépendamment un atome d'hydrogène, un groupe -(CH₂)ₐ-CONR₁R₂, (CH₂)a-NR₁-COR₂, ou 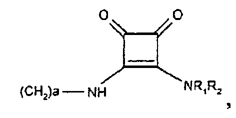 avec :
a = 1, 2 ou 3 ;
chaque R₁, R₂ représente indépendamment un atome H, une chaîne alkyle ou alcényle en C₇-C₃₀, substituée ou non substituée, linéaire ou ramifiée ou cyclique, ou un groupe 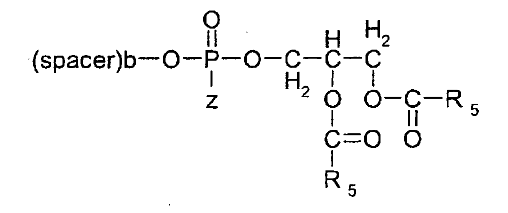 avec b = 0, 1 ou 2 , Z représente O⁻ ou OH, R₅ représente un groupe saturé ou insaturé d'au moins 6 atomes de carbone avantageusement en C₆ à C₃₀, éventuellement substitué, et spacer représente un groupe CH₂CH₂ ou polyalkylèneglycol ;
4) X₃, X₄ et X₅, identiques ou différents ont la même signification que X₁ ou X₂ à la condition que les X₁, X₂, X₃, X₄ et X₅ ne soient pas tous H et qu'au moins un des X₁, X₂, X₃, X₄, X₅ soit un groupe lipophile choisi parmi :
• un groupe -(CH₂)ₐ-CONR₁R₂ , -(CH₂)ₐ-NR₁COR₂ ou 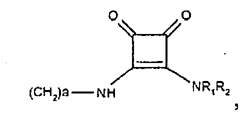 avec ; chaque R₁, R₂ représentent indépendamment une chaîne alkyle ou alcényle en C₇ à C₃₀ linéaire ou ramifié ou cyclique éventuellement interrompue par un ou deux atomes ou groupes O, S, NH ou NR₃ où R₃ est un alkyle en C₁-C_{3;} ou un groupe 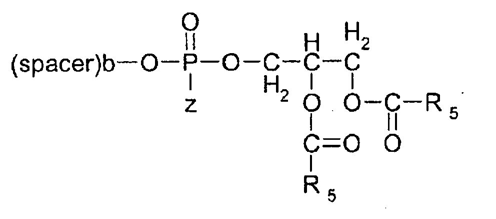 avec b = 0, 1 ou 2 , Z représente O⁻ ou OH, R₅ représente un groupe saturé ou insaturé d'au moins 6 atomes de carbone avantageusement en C₆ à C₃₀, éventuellement substitué, et spacer représente un groupe CH₂CH₂ ou polyalkylèneglycol ;
• un groupe 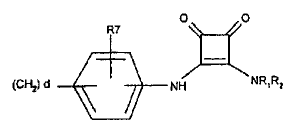
ou 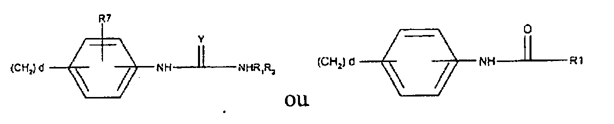 avec :
d compris entre 0 et 3 ;
Y choisi parmi O et S ;
R₇ choisi parmi H, OH, CH₃, OCH₃ ;
chaque R₁, R₂ représentent indépendamment une chaîne alkyle ou alcényle en C₇ à C₃₀ linéaire ou ramifié ou cyclique éventuellement interrompue par un ou deux atomes ou groupes O, S, NH ou NR₃ où R₃ est un alkyle en C₁-C₃ ; ou un groupe 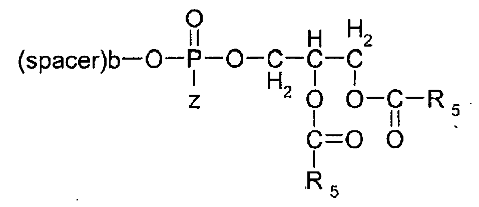 avec b = 0, 1 ou 2, Z représente O⁻ ou OH, R₅ représente un groupe saturé ou insaturé d'au moins 6 atomes de carbone avantageusement en C₆ à C₃₀, éventuellement substitué, et spacer représente un groupe CH₂CH₂ ou polyalkylèneglycol ;
ou X₃, X₄ et X₅, identiques ou différents, représentent 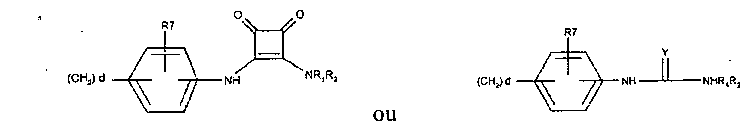 ou 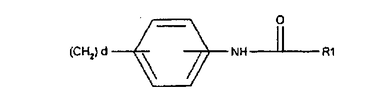 d compris entre 0 et 3 ;
Y choisi parmi O et S ;
R₇ choisi parmi H, OH, CH₃, OCH₃ ;
R₁ et R₂ sont tels que définis ci-dessus ;
5) A₁, A₂, B₁, B₂, C₁, C₂, D₁, D₂, E₁, E₂, F₁, F₂, représentent indépendamment les uns des autres un groupe H ou CH₃ ou cyclohexyle ;
6) K représente C ou N ou CH ou NH ou N⁺R₄ avec R₄ un groupe en C₁-C₆ choisi parmi alkyle, benzyle ou benzyle substitué ;
7) G représente rien ou O ou NHR₄, R₄ est tel que défini ci-dessus et ses sels pharmaceutiquement acceptables.

3. Composé selon la revendication 1 ou 2 dans lequel R₁ et/ou R₂ représentent une chaîne alkyle ou alcényle en C₇-C₂₄, de préférence en C₈-C₁₈.

4. Complexe d'un ion de métal paramagnétique et d'un chélate organique de formule (I) selon les revendications 1 à 3.

5. Complexe selon la revendication 4 dans lequel l'ion métallique est un lanthanide de nombre atomique 58-70 ou métal de transition de nombre atomique 21-29, 42 ou 44.

6. Complexe selon la revendication 4 ou 5 dans lequel l'ion métallique est choisi parmi Gd(III), Mn(II), fer, dysprosium.

7. Composition lipidique physiologiquement acceptable destinée à l'imagerie IRM comprenant au moins un complexe selon les revendications 4 à 6.

8. Composition selon la revendication 7 sous forme d'émulsion, de liposomes, de micelles.

9. Composition selon les revendications 7 ou 8 comprenant de l'eau, une phase lipidique dispersée, éventuellement un fluorocarbone, et au moins un complexe selon les revendications 4 à 6.

10. Procédé de préparation d'une composition selon la revendication 7 à 9 comprenant :
- la préparation d'un mélange comprenant un complexe selon les revendications 4 à 6 et une phase aqueuse ;
- l'agitation du mélange de manière à obtenir une dispersion homogène des constituants.

11. Composé de formule (II) 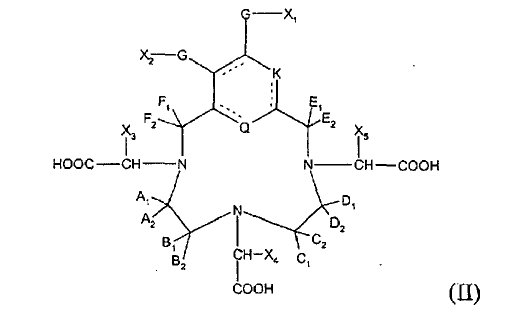 dans laquelle :
1) --- représente une simple ou double liaison ;
2) A₁, A₂, B₁, B₂, C₁, C₂, D₁, D₂, E₁, E₂, F₁, F₂, Q, K, R₇ ont la même signification que dans les revendications précédentes, G représente rien ; et X₁, X₂, X₃, X₄ ou X₅ identiques ou différents, représentent indépendamment (CH₂)a-NH₂ ou (CH₂)a-NO₂ ou 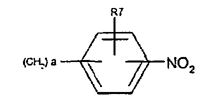 avec a=1, 2 ou 3 ou
A₁, A₂, B₁, B₂, C₁, C₂, D₁, D₂, E₁, E₂, F₁, F₂, Q, K, R₇ ont la même signification que dans les revendications précédentes, G représente O ou NHR₄ dans lequel R₄ a la même signification que dans les revendications précédentes et X₁, X₂, X₃, X₄ ou X₅ identiques ou différents représentent indépendamment (CH₂)a-CO₂H, ou 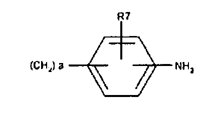 ou (CH2)a-NH2 ou (CH2)a-NO2 ou 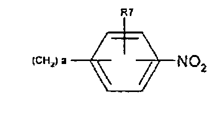
avec a=1, 2 ou 3.

## Claims

1. A compound of formula (I) 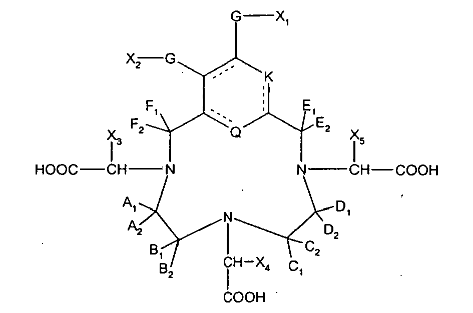 wherein:
1) --- represents a single or double bond;
2) Q represents a nitrogen atom or an NH radical;
3) X₁, X₂, X₃, X₄ and X₅, either identical or different, independently represent
3.1) a hydrogen atom;
3.2) a -(CH₂)ₐ-CONR₁R₂, -(CH₂)ₐ-NR₁COR₂ or 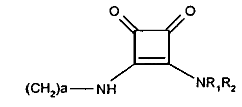 group, with;
each R₁, R₂ independently represents an H atom; a substituted or unsubstituted, linear or branched or cyclic C₇-C₃₀ alkyl or alkenyl chain optionally interrupted by O, NH, NR₃ or S, wherein R₃ is a C₁-C₃ alkyl; or a group 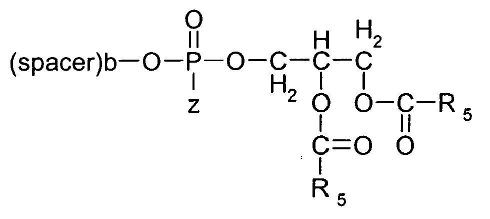 with b = 0, 1 or 2, Z represents O⁻ or OH, R₅ represents an optionally substituted, saturated or unsaturated group with at least 6 carbon atoms, advantageously a C₆-C₃₀ group and spacer represents a CH₂CH₂ or polyalkylene glycol group;
3.3) a group 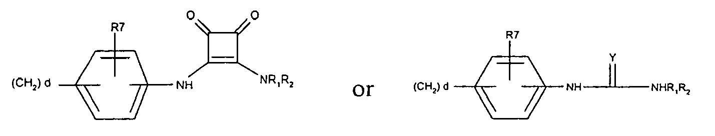 or 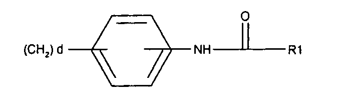 with:
d comprised between 0 and 3;
Y selected from O and S;
R₇ selected from H, OH, CH₃ or OCH_{3;}
R₁ and R₂ are as defined above;
with the proviso that X₁, X₂, X₃, X₄ and X₅ are not all H and that at least one of X₁, X₂, X₃, X₄ and X₅ is a liphophilic group selected from:
• a -(CH₂)ₐ-CONR₁R₂, -(CH₂)ₐ-NR₁COR₂ or 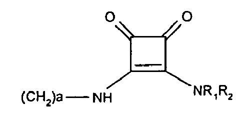 group, with;
each R₁, R₂ independently represents a linear or branched or cyclic C₇-C₃₀ alkyl or alkenyl chain optionally interrupted by one or two O, S atoms or NH, NR₃ groups, wherein R₃ is a C₁-C₃ alkyl; or a group 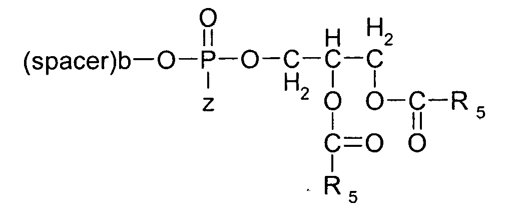 with b = 0, 1 or 2, Z represents O⁻ or OH, R₅ represents an optionally substituted, saturated or unsaturated group with at least 6 carbon atoms, advantageously a C₆-C₃₀ group and spacer represents a CH₂CH₂ or polyalkylene glycol group;
• a group 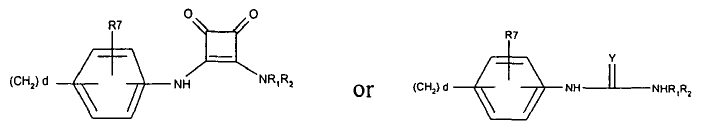 or 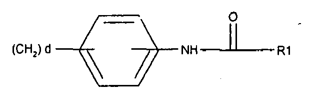 with:
d comprised between 0 and 3;
Y selected from O and S;
R₇ selected from H, OH, CH₃ or OCH₃;
each R₁, R₂ independently represents a linear or branched or cyclic C₇-C₃₀ alkyl or alkenyl chain optionally interrupted by one or two O, S atoms or NH, NR₃ groups, wherein R₃ is a C₁-C₃ alkyl; or a group 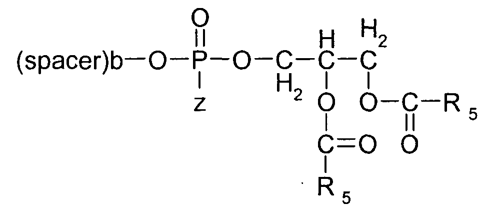 with b = 0, 1 or 2, Z represents O⁻ or OH, R₅ represents an optionally substituted, saturated or unsaturated group with at least 6 carbon atoms, advantageously a C₆-C₃₀ group, and spacer represents a CH₂CH₂ or polyalkylene glycol group;
4) A₁, A₂, B₁, B₂, C₁, C₂, D₁, D₂, E₁, E₂, F₁, F₂ represent, independently of each other, an H or CH₃ or cyclohexyl group;
5) K represents C or N or CH or NH or N⁺R₄ with R₄ a C₁-C₆ group selected from alkyl, benzyl or substituted benzyl;
6) G represents nothing or represents O or NHR₄, with R₄ as defined above; and its pharmaceutically acceptable salts.

2. A compound of formula (I) 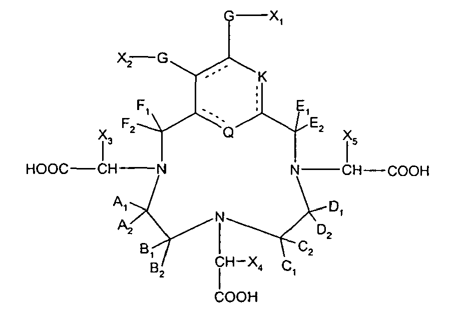 wherein:
1) --- represents a single or double bond
2) Q represents a nitrogen atom or an NH radical;
3) X₁ or X₂, either identical or different, independently represent a hydrogen atom, a -(CH₂)ₐ-CONR₁R₂, -(CH₂)ₐ-NR₁-COR₂ or 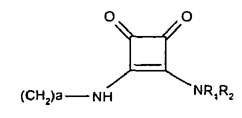 group, with:
a = 1, 2 or 3;
each R₁, R₂ independently represents an H atom, a substituted or unsubstituted, linear or branched or cyclic C₇-C₃₀ alkyl or alkenyl chain, or a group 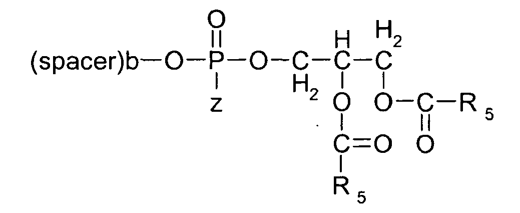 with b = 0, 1 or 2, Z represents O⁻ or OH, R₅ represents an optionally substituted, saturated or unsaturated group with at least 6 carbon atoms, advantageously a C₆-C₃₀ group, and spacer represents a CH₂CH₂ or polyalkylene glycol group;
4) X₃, X₄ and X₅, either identical or different, have the same meaning as X₁ or X₂ with the proviso that X₁, X₂, X₃, X₄ and X₅ are not all H and at least one of X₁, X₂, X₃, X₄ and X₅ is a lipophilic group selected from:
• a -(CH₂)ₐ-CONR₁R₂, (CH₂)ₐ-NR₁-COR₂ or 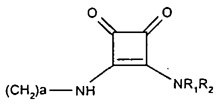 group, with:
each R₁, R₂ independently represents a substituted or unsubstituted, linear or branched or cyclic C₇-C₃₀ alkyl or alkenyl chain, optionally interrupted by one or two O, S atoms or NH, NR₃ groups, wherein R₃ is a C₁-C₃ alkyl; or a group 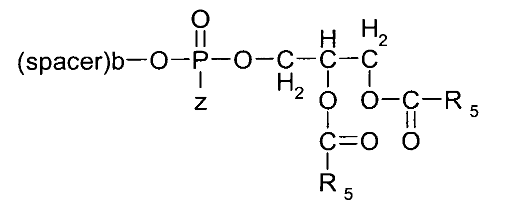 with b = 0, 1 or 2, Z represents O⁻ or OH, R₅ represents an optionally substituted, saturated or unsaturated group with at least 6 carbon atoms, advantageously a C₆-C₃₀ group, and spacer represents a CH₂CH₂ or polyalkylene glycol group;
• a group 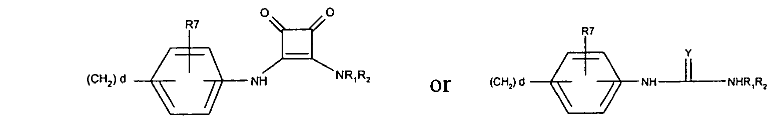 or 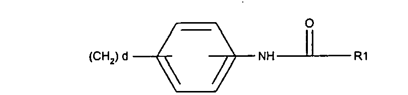 with:
d comprised between 0 and 3;
Y selected from O and S;
R₇ selected from H, OH, CH₃ or OCH_{3;}
each R₁, R₂ independently represents a substituted or unsubstituted, linear or branched or cyclic C₇-C₃₀ alkyl or alkenyl chain, optionally interrupted by one or two O, S atoms or NH, NR₃ groups, wherein R₃ is a C₁-C₃ alkyl; or a group 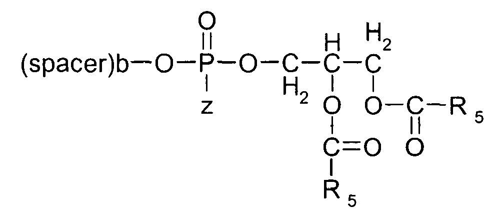 with b = 0, 1 or 2, Z represents O⁻ or OH, R₅ represents an optionally substituted, saturated or unsaturated group with at least 6 carbon atoms, advantageously a C₆-C₃₀ group, and spacer represents a CH₂CH₂ or polyalkylene glycol group;
or X₃, X₄ and X₅, either identical or different, represent 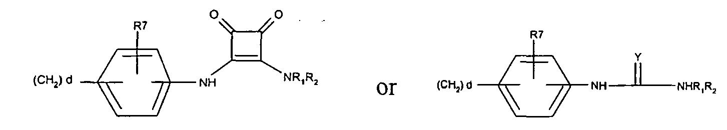 or 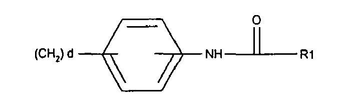 d comprised between 0 and 3;
Y selected from O and S;
R₇ selected from H, OH, CH₃ or OCH_{3;}
R₁ and R₂ are as defined above;
5) A₁, A₂, B₁, B₂, C₁, C₂, D₁, D₂, E₁, E₂, F₁, F₂ represent, independently of each other, an H or CH₃ or cyclohexyl group;
6) K represents C or N or CH or NH or N⁺R₄ with R₄ a C₁-C₆ group selected from alkyl, benzyl or substituted benzyl;
7) G represents nothing or represents O or NHR₄, with R₄ as defined above;
and its pharmaceutically acceptable salts.

3. The compound according to claim 1 or 2, wherein R₁ and/or R₂ represent a C₇-C₂₄, preferably C₈-C₁₈, alkyl or alkenyl chain.

4. A complex of a paramagnetic metal ion and of an organic chelate of formula (I) according to claims 1 to 3.

5. The complex according to claim 4, wherein the metal ion is a lanthanide of atomic number 58-70 or a transition metal of atomic number 21-29, 42 or 44.

6. The complex according to claim 4 or 5, wherein the metal ion is selected from Gd(III), Mn(II), iron or dysprosium.

7. A physiologically acceptable lipid composition intended for MRI imaging comprising at least one complex according to claims 4 to 6.

8. The composition according to claim 7, in the form of an emulsion of liposomes or of micelles.

9. The composition according to claims 7 or 8, comprising water, a dispersed lipid phase, optionally a fluorocarbon and at least one complex according to claims 4 to 6.

10. A method for preparing a composition according to claims 7 to 9, comprising:
- preparing a mixture comprising a complex according to claims 4 to 6 and an aqueous phase;
- stirring the mixture so as to obtain a homogeneous dispersion of the constituents.

11. A compound of formula (II) 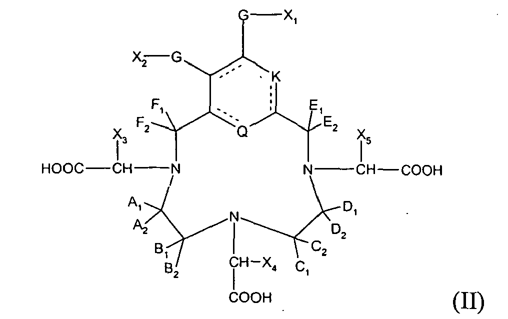 wherein:
1) --- represents a single or double bond;
2) A₁, A₂, B₁, B₂, C₁, C₂, D₁, D₂, E₁, E₂, F₁, F₂, Q, K and R₇ have the same meaning as in the preceding claims, G represents nothing; and X₁, X₂, X₃, X₄ or X₅, either identical or different, independently represent (CH₂)ₐ-NH₂ or (CH₂)ₐ-NO₂ or 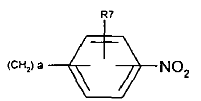 with a=1, 2 or 3 or
A₁, A₂, B₁, B₂, C₁, C₂, D₁, D₂, E₁, E₂, F₁, F₂, Q, K and R₇ have the same meaning as in the preceding claims, G represents O or NHR₄, wherein R₄ has the same meaning as in the preceding claims, and X₁, X₂, X₃, X₄ or X₅, either identical or different, independently represent (CH₂)ₐ-CO₂H or 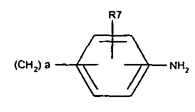 or (CH₂)ₐ-NH₂ or (CH₂)ₐ-NO₂ or 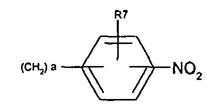 with a=1, 2 or 3.

## Patentansprüche

1. Formelverbindung (I) 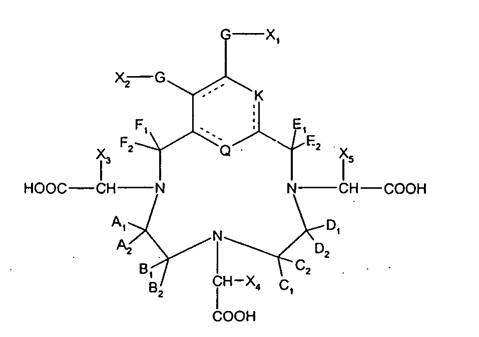 wobei:
1) --- einer Einfach- oder Doppelverbindung entspricht;
2) Q ein Stickstoffatom- oder ein NH-Radikal darstellt;
3) X₁, X₂, X₃, X₄ und X₅, die identisch oder unterschiedlich sind, unabhängig:
3.1) ein Wasserstoffatom darstellen;
3.2) eine -(CH₂)ₐ-CONR₁R₂-, -(CH₂)ₐ-NR₁COR₂- oder 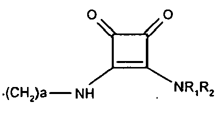 -Gruppe darstellen, wobei R₁ oder R₂ jeweils unabhängig ein H-Atom; eine substituierte oder nicht substituierte, lineare oder verzweigte bzw. zyklische Alkyl- oder Alkenylkette darstellen, die ggf. durch O, NH, NR₃ oder S, unterbrochen wird, wobei R₃ ein C₁-C₃-Alkyl ist, oder eine Gruppe 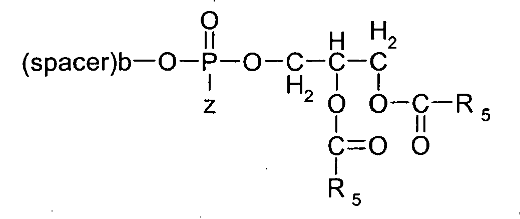 mit b = = 0, 1 oder 2 darstellen, Z stellt O⁻ oder OH, R₅ eine gesättigte bzw. ungesättigte, ggf. substituierte Gruppe von zumindest 6 Kohlenstoffatomen nach einer vorteilhaften Ausführung in C₆ bis C₃₀ und Spacer eine CH₂CH₂- oder Polyalkylenglykol-Gruppe dar;
3.3) eine Gruppe 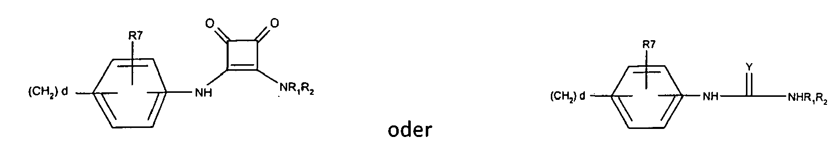 oder 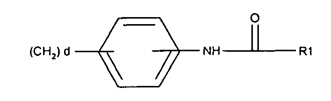 darstellen, wobei:
d zwischen 0 und 3 liegt;
Y aus O und S ausgewählt wird;
R₇ aus H, OH, CH₃ oder OCH₃ ausgewählt wird;
R₁ und R₂ der nachstehenden Definition entsprechen,
jedoch unter der Bedingung, dass X₁, X₂, X₃, X₄ und X₅ nicht in ihrer Gesamtheit H sind und zumindest einer der X₁, X₂, X₃, X₄, X₅ eine lipophile Gruppe ist, die aus den folgenden Gruppen ausgewählt wird:
- einer -(CH₂)ₐ-CONR₁R₂ -, -(CH₂)ₐ-NR₁COR₂- oder 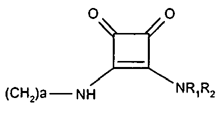 -Gruppe, wobei R₁ oder R₂ jeweils unabhängig ein H-Atom; eine substituierte oder nicht substituierte, lineare oder verzweigte bzw. zyklische Alkyl- oder Alkenylkette darstellt, die ggf. durch O, NH, NR₃ oder S, unterbrochen wird, wobei R₃ ein C₁-C₃-Alkyl ist, oder einer Gruppe 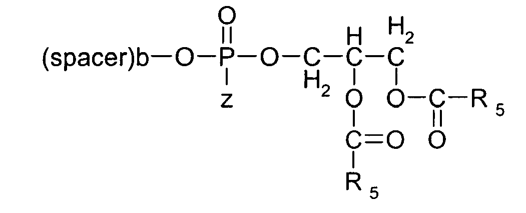 mit b = 0, 1 oder 2, Z stellt O⁻ oder OH, R₅ eine gesättigte bzw. ungesättigte, ggf. substituierte Gruppe von zumindest 6 Kohlenstoffatomen nach einer vorteilhaften Ausführung in C₆ bis C₃₀ und Spacer eine CH₂CH₂- oder Polyalkylenglykol-Gruppe dar;
- einer Gruppe 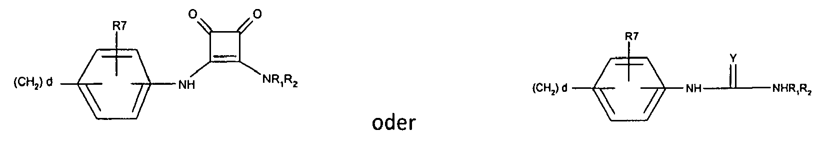 oder 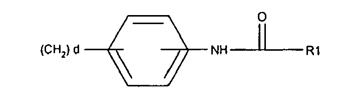 wobei:
d zwischen 0 und 3 liegt;
Y aus O und S ausgewählt wird;
R₇ aus H, OH, CH₃ oder OCH₃ ausgewählt wird;
wobei R₁ und R₂ jeweils unabhängig eine lineare oder verzweigte oder zyklische Alkyl- oder Alkenylkette in C₆ bis C₃₀ darstellt, die ggf. durch ein oder zwei O, S, NH oder NR₃-Atome oder - Gruppen unterbrochen wird, wobei oder R₃ ein Alkyl in C₁ - C₃ ist, oder einer Gruppe 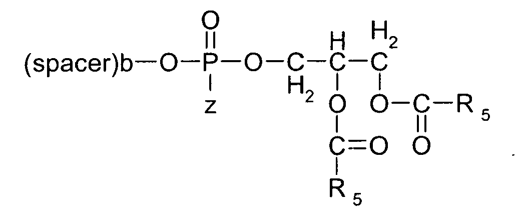 mit b = 0, 1 oder 2, wobei Z stellt O⁻ oder OH, R₅ eine gesättigte bzw. ungesättigte, ggf. substituierte Gruppe von zumindest 6 Kohlenstoffatomen nach einer vorteilhaften Ausführung in C₆ bis C₃₀ und Spacer eine CH₂CH₂- oder Polyalkylenglykol-Gruppe dar;
4) A₁, A₂, B₁, B₂, C₁, C₂, D₁, D₂, E₁, E₂, F₁ und F₂ unabhängig voneinander eine H- oder CH- oder Cyclohexyl-Gruppe darstellen;
5) K C oder N oder CH oder NH oder N⁺R₄ mit R₄ in C₁-C₆ eine Gruppe darstellt, die aus Alkyl, Benzyl oder substituierten Benzyl ausgewählt wird;
6) G nicht dargestellt wird oder O oder NHR₄, R₄ wie oben definiert und ihre annehmbaren pharmazeutischen Salzen darstellt.

2. Formelverbindung (I) 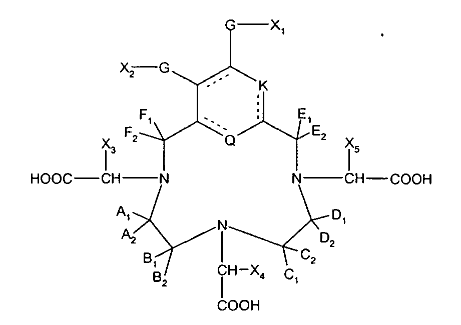 wobei:
1) --- einer Einfach- oder Doppelverbindung entspricht;
2) Q ein Stickstoffatom- oder ein NH-Radikal darstellt;
3) X₁ oder X₂, die identisch oder unterschiedlich sind, unabhängig voneinander ein Wasserstoffatom, eine -(CH₂)ₐ-CONR₁R₂, -(CH₂)ₐ-NR₁-COR₂ - oder 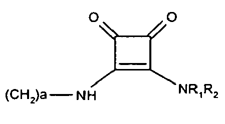 -Gruppe darstellen, wobei:
a=1, 2 oder 3;
R₁ oder R₂ jeweils unabhängig ein Atom H, eine substituierte oder nicht substituierte, lineare oder verzweigte oder zyklische Alkyl- oder Alkenylkette in C₇-C₃₀ oder eine Gruppe 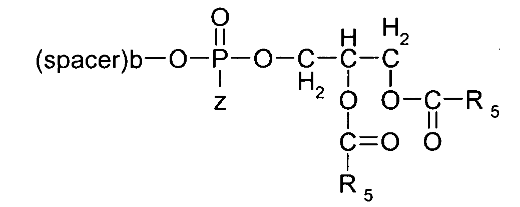 darstellt, wobei b = 1, 2 oder 3, Z stellt O⁻ oder OH, R₅ eine gesättigte oder ungesättigte, ggf. substituierte Gruppe von zumindest 6 Kohlenstoffatomen nach einer vorteilhaften Ausführung in C₆ bis C₃₀ und Spacer eine CH₂CH - oder Polyalkylenglykol-Gruppe dar;
4) X₃, X₄ und X₅, die identisch oder unterschiedlich sind, dieselbe Bedeutung haben wie X₁ oder X₂, sofern X₁, X₂, X₃, X₄ und X₅ nicht in ihrer Gesamtheit H sind und zumindest einer der X₁, X₂, X₃, X₄ und X₅ eine lipophile Gruppe ist, die aus den folgenden Gruppen ausgewählt wird:
- einer -(CH₂)ₐ-CONR₁R₂-, -(CH₂)ₐ-NR₁COR₂- oder 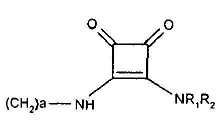
- Gruppe, wobei R₁, R₂ in C₇ bis C₃₀ jeweils unabhängig eine lineare oder verzweigte bzw. zyklische Alkyl- oder Alkenylkette darstellt, die ggf. durch ein oder zwei O-, S, NH-, NR₃-Atome oder Gruppen unterbrochen wird, wobei R₃ ein C₁-C₃-Alkyl ist, oder einer Gruppe 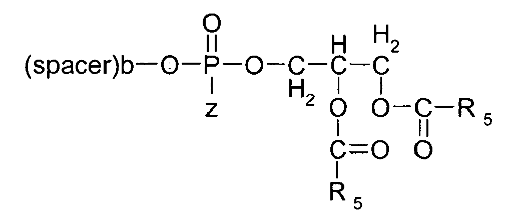 mit b = 0, 1 oder 2, Z stellt O⁻ oder OH, R₅ eine gesättigte bzw. ungesättigte, ggf. substituierte Gruppe von zumindest 6 Kohlenstoffatomen nach einer vorteilhaften Ausführung in C₆ bis C₃₀ und Spacer eine CH₂CH₂- oder Polyalkylenglykol-Gruppe dar;
- einer Gruppe 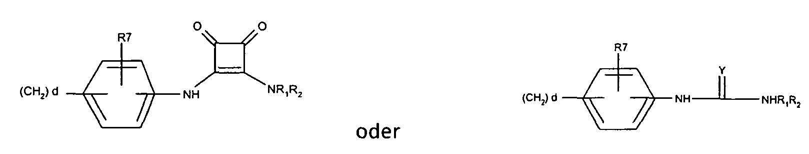 oder 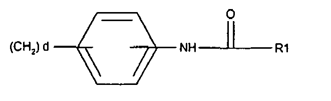 wobei
d zwischen 0 und 3 beträgt;
Y aus 0 und S ausgewählt wird;
R₇ aus H, OH, CH₃ oder OCH₃ ausgewählt wird;
R₁, R₂ in C₇ bis C₃₀ jeweils unabhängig eine lineare oder verzweigte bzw. zyklische Alkyl- oder Alkenylkette darstellt, die ggf. durch ein oder zwei O-, S, NH-, NR₃-Atome oder Gruppen unterbrochen wird, wobei R₃ ein C₁-C₃-Alkyl ist, oder einer Gruppe 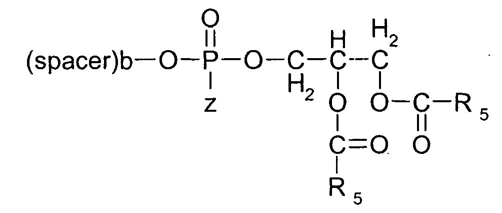 mit b = 0, 1 oder 2, Z stellt O⁻ oder OH, R₅ eine gesättigte bzw. ungesättigte, ggf. substituierte Gruppe von zumindest 6 Kohlenstoffatomen nach einer vorteilhaften Ausführung in C₆ bis C₃₀ und Spacer eine CH₂CH₂- oder Polyalkylenglykol-Gruppe dar;
oder X₃, X₄ und X₅, die identisch oder unterschiedlich sind, 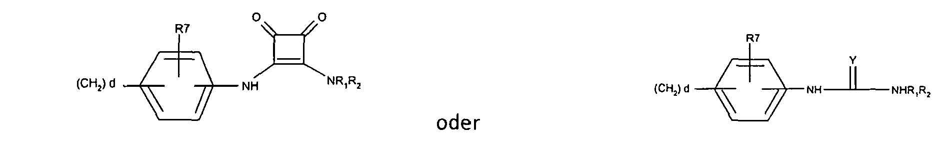 oder 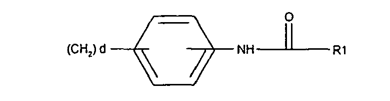 darstellen,
d zwischen 0 und 3 beträgt;
Y aus O und S ausgewählt wird;
R₇ aus H, OH, CH₃ oder OCH₃ ausgewählt wird;
R₁, R₂ weiter oben definiert werden.
5) A₁, A₂, B₁, B₂, C₁, C₂, D₁, D₂, E₁, E₂, F₁ und F₂ unabhängig voneinander eine H- oder CH₃- oder Cyclohexyl-Gruppe darstellen;
6) K C oder N oder CH oder NH oder N⁺R₄ mit R₄ in C₁-C₆ eine Gruppe darstellt, die aus Alkyl, Benzyl oder substituierten Benzyl ausgewählt wird;
7) G nicht dargestellt wird oder O oder NHR₄, R₄ wie oben definiert und ihre annehmbaren pharmazeutischen Salzen darstellt.

3. Verbindung nach Anspruch 1 oder 2, bei der R₁ und/oder R₂ in C₇-C₂₄ und nach einer bevorzugten Ausführung in C₈-C₁₈ eine Alkyl- oder Alkenylkette darstellen.

4. Komplex aus einem paramagnetischen Metallion und einem organischen Formelchelat (I) nach einem der Ansprüche 1bis 3.

5. Komplex nach Anspruch 4, bei dem das Metallion ein Lanthamid mit Kernladungszahl 58-70 oder ein Übergangsmetall mit Kernladungszahl 21-29, 42 oder 44 ist.

6. Komplex nach Anspruch 4 oder 5, bei dem das Metallion aus Gd(III), Mn(II), Iron oder Dysprosium ausgewählt wird.

7. Physiologisch verträgliche Fettverbindung für Kernspintomographie, die zumindest einen Komplex nach Anspruch 4 bis 6 umfasst.

8. Verbindung nach Anspruch 7 in Emulsions-,Liposom-,Mizelleform.

9. Verbindung nach Anspruch 7 oder 8, die Wasser, eine dispergierte Fettphase, ggf. einen Fluorkohlenstoff und zumindest einen Komplex nach Anspruch 4 bis 6 umfasst.

10. Zubereitungsverfahren einer Verbindung nach Anspruch 7 bis 9, die folgendes umfasst:
- die Zubereitung einer Mischung, die einen Komplex nach Anspruch 4 bis 6 und eine wässrige Phase umfasst;
- das Schütteln der Mischung derart, dass eine homogene Streuung der Bestandteile erlangt wird.

11. Formelverbindung (II) 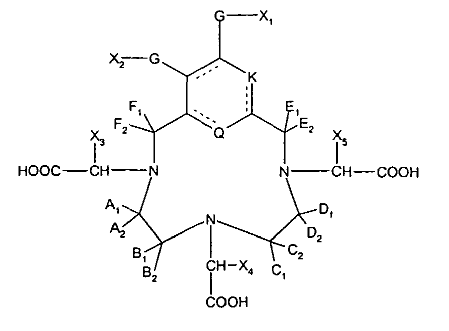 wobei:
1) --- eine Einfach- oder Doppelverbindung darstellt;
2) A₁, A₂, B₁, B₂, C₁, C₂, D₁, D₂, E₁, E₂, F₁, F₂, Q, K und R₇ dieselbe Bedeutung wie in den vorstehenden Ansprüchen haben, G nicht dargestellt wird und X₁, X₂, X₃, X₄ oder X₅, die identisch oder verschieden sind, unabhängig (CH₂)ₐ-NH₂ oder (CH₂)ₐ-NO₂ oder 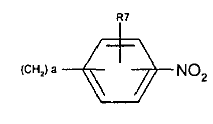 mit a=1, 2 oder 3 darstellen oder
A₁, A₂, B₁, B₂, C₁, C₂, D₁, D₂, E₁, E₂, F₁, F₂, Q, K und R₇ dieselbe Bedeutung wie in den vorstehenden Ansprüchen haben; G O oder NHR₄ darstellt, wobei R₄ die dieselbe Bedeutung wie in den vorstehenden Ansprüchen hat und X₁, X₂, X₃, X₄ oder X₅, die identisch oder verschieden sind, unabhängig (CH₂)ₐ-CO₂H darstellen,
oder 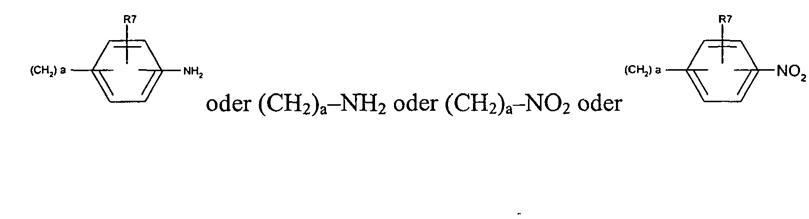 mit a = 1, 2 oder 3.
